(19) Europäisches Patentamt
European Patent Office
Office européen des brevets

(11) Veröffentlichungsnummer: **0 155 903**
**B1**

(12) # EUROPÄISCHE PATENTSCHRIFT

(45) Veröffentlichungstag der Patentschrift: **23.08.89**

(51) Int. Cl.⁴: **C 07 D 457/14**, A 61 K 31/475

(21) Anmeldenummer: **85810015.9**

(22) Anmeldetag: **22.01.85**

(54) **Indolophenanthridine, ihre Herstellung und Verwendung.**

(30) Priorität: **25.01.84 DE 3402392**

(43) Veröffentlichungstag der Anmeldung: **25.09.85 Patentblatt 85/39**

(45) Bekanntmachung des Hinweises auf die Patenterteilung: **23.08.89 Patentblatt 89/34**

(84) Benannte Vertragsstaaten: **AT BE CH DE FR GB IT LI LU NL SE**

(56) Entgegenhaltungen: **GB-A-2 093 452**

(73) Patentinhaber: **SANDOZ AG, Lichtstrasse 35, CH-4002 Basel (CH)**
(84) Benannte Vertragsstaaten: **BE CH FR GB IT LI LU NL SE**

(73) Patentinhaber: **SANDOZ- PATENT- GMBH, Humboldtstrasse 3, D-7850 Lörrach (DE)**
(84) Benannte Vertragsstaaten: **DE**

(73) Patentinhaber: **SANDOZ- ERFINDUNGEN Verwaltungsgesellschaft m.b.H., Brunner Strasse 59, A-1235 Wien (AT)**
(84) Benannte Vertragsstaaten: **AT**

(72) Erfinder: **Hagenbach, Alexander, Hangelimattweg 111, CH- 4148 Pfeffingen (CH)**
Erfinder: **Seiler, Max Peter, Clarastrasse 21,, CH-4058 Basel (CH)**
Erfinder: **Wüthrich, Hans Jürg, Gurtenstrasse 53, CH- 3122 Kehrsatz (CH)**

**EP 0 155 903 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

## Beschreibung

Gegenstand der vorliegenden Erfindung sind Indolophenanthridine, Verfahren zu deren Herstellung, pharmazeutische Zusammensetzungen, die diese Indolophenanthridine enthalten, sowie die Anwendung dieser Verbindungen bei der therapeutischen Behandlung.

Insbesondere werden Verbindungen der Formel I umfasst,

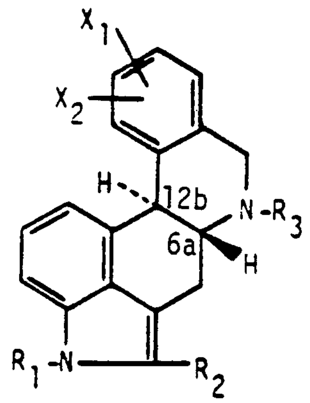

I

worin

$R_1$ und $R_3$ unabhängig voneinander Wasserstoff, $(C_{1-4})$Alkyl, $(C_{3-5})$Alkenyl, wobei die Doppelbindung nicht zu dem Stickstoffatom benachbart ist, $(C_{3-6})$Cycloalkyl$(C_{1-3})$alkyl, im Phenylring gegebenenfalls durch $(C_{1-4})$Alkyl, $(C_{1-4})$Alkoxy, Hydroxy oder Halogen substituiertes Phenyl$(C_{1-3})$alkyl oder 2- oder 3-Furyl$(C_{1-3})$alkyl bedeuten,

$R_2$ für Wasserstoff, Chlor, Brom oder Methyl steht, und

$X_1$ und $X_2$ unabhängig voneinander für Wasserstoff, $(C_{1-4})$Alkyl, $(C_{1-4})$Alkoxy, Hydroxy, Halogen oder Trifluormethyl stehen,

in Form der freien Basen oder von Säureadditionssalzen. Diese Verbindungen werden im folgenden als neue Verbindungen bezeichnet.

Die neuen Verbindungen zeichnen sich durch zwei asymmetrische Kohlenstoffatome aus, in Stellung 6a und 12b. Sie können deshalb in racemischer oder optisch aktiver Form vorliegen. Gegenstand der Erfindung sind sowohl die Racemate wie auch die Enantiomere.

Die neuen Verbindungen können in freier Form oder als Säureadditionssalze vorliegen. Sowohl die freie Form wie auch die Salzformen sind Gegenstand der Erfindung. Beispiele von Säureadditionssalzformen sind die Hydrochloride, die Hydrobromide und die Hydrogenfumarate.

Sofern in den Verbindungen der Formel I vorstehend definierte Alkyl- oder Alkoxygruppen enthalten sind, besitzen diese vorzugsweise 1 oder 2 Kohlenstoffatome und stellen insbesondere Methyl oder Methoxy dar.

Unter Halogen werden Halogenatome mit einer Atomzahl von 9 bis 53 gemeint, insbesondere Chlor und Brom.

Stehen $R_1$ und/oder $R_3$ für im Phenylring substituiertes Phenyl$(C_{1-3})$alkyl, so kann diese Gruppe mono-, di- oder trisubstituiert sein.

In der Formel I werden die folgenden Bedeutungen und deren Kombinationen bevorzugt:

$R_1$ bedeutet Wasserstoff oder $(C_{1-4})$Alkyl;

$R_2$ besitzt obige Bedeutung.

$R_3$ bedeutet Wasserstoff, $(C_{1-4})$Alkyl, gegebenenfalls durch $(C_{1-4})$Alkoxy monosubstituiertes Benzyl oder 2-Furylmethyl;

$X_1$ besitzt obige Bedeutung;

$X_2$ steht für Wasserstoff.

Die Erfindung umfasst z. B. eine Gruppe von Verbindungen der Formel I, worin

$R_1$ und $R_3$ unabhängig voneinander Wasserstoff, $(C_{1-4})$Alkyl, $(C_{3-5})$Alkenyl, wobei die Doppelbindung nicht zu dem Stickstoffatom benachbart ist, $(C_{3-6})$Cycloalkyl$(C_{1-3})$alkyl, im Phenylring gegebenenfalls durch Methyl, Methoxy, Hydroxy oder Halogen monosubstituiertes Phenyl$(C_{1-3})$alkyl oder 2- oder 3-Furyl$(C_{1-3})$alkyl bedeuten,

$R_2$ für Wasserstoff, Chlor, Brom oder Methyl steht, und

$X_1$ und $X_2$ unabhängig voneinander für Wasserstoff, $(C_{1-4})$Alkyl, $(C_{1-4})$Alkoxy, Hydroxy, Halogen oder Trifluormethyl stehen,

in Form der freien Basen oder von Säureadditionssalzen.

Gemäss der vorliegenden Erfindung gelangt man zu den Verbindungen der Formel I und ihren Säureadditionssalzen, indem man Verbindungen der Formel II,

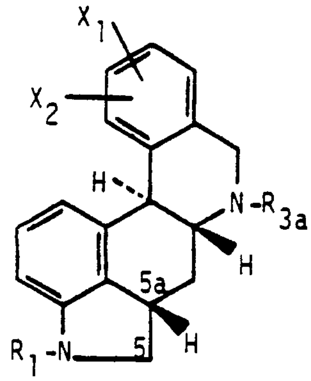

II

worin

$R_1$, $X_1$ und $X_2$ obige Bedeutung besitzen und $R_{3a}$ die für $R_3$ angegebene Bedeutung mit Ausnahme von Wasserstoff besitzt, in 5,5a-Stellung des Indolophenanthridingerüstes oxydiert, die erhaltenen Verbindungen der Formel Ia,

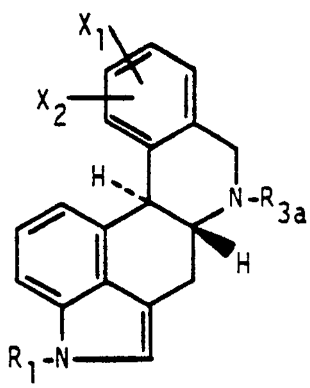

Ia

worin

$R_1$, $R_{3a}$, $X_1$ und $X_2$ obige Bedeutung besitzen, zum Beispiel mittels Alkylierungs-, Desalkylierungs- und Halogenierungsmethoden gewünschtenfalls in die übrigen Verbindungen der Formel I überführt und die erhaltenen Verbindungen der Formel I als freie Basen oder als Säureadditionssalze gewinnt.

So werden aus den Verbindungen der Formel Ia die übrigen Verbindungen der Formel I hergestellt, indem man die folgenden Herstellungsverfahren allein oder in Kombination anwendet:

a) Zur Herstellung von Verbindungen der Formel Ib,

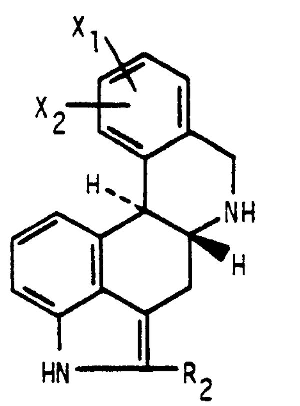

Ib

worin

$R_2$, $X_1$ und $X_2$ obige Bedeutung besitzen, werden Verbindungen der Formel I',

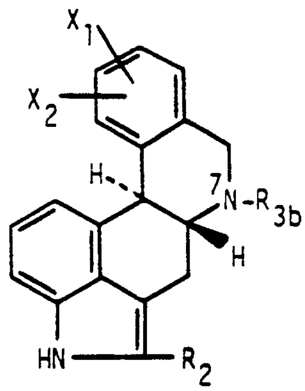

I'

worin

$R_2$, $X_1$ und $X_2$ obige Bedeutung besitzen und $R_{3b}$ Methyl oder Benzyl bedeutet, in 7-Stellung desalkyliert;

b) zur Herstellung von Verbindungen der Formel Ic,

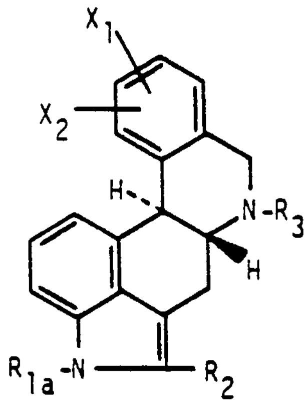

Ic

worin

$R_2$, $R_3$, $X_1$ und $X_2$ obige Bedeutung besitzen und $R_{1a}$ die für $R_1$ angegebene Bedeutung mit Ausnahme von Wasserstoff besitzt, werden Verbindungen der Formel I'',

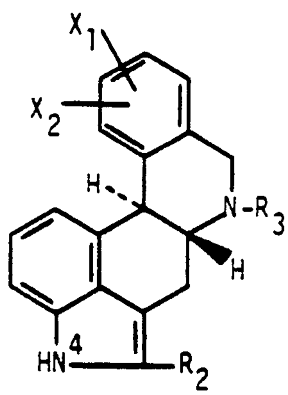

I''

worin

$R_2$, $R_3$, $X_1$ und $X_2$ obige Bedeutung besitzen, in 4-Stellung alkyliert;

c) zur Herstellung von Verbindungen der Formel Id,

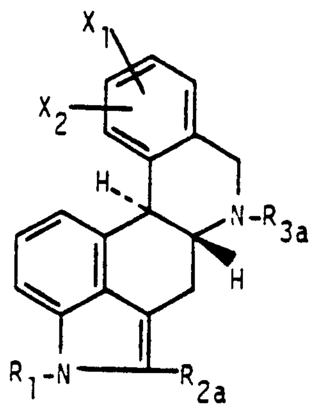

Id

worin

$R_1$, $R_{3a}$, $X_1$ und $X_2$ obige Bedeutung besitzen und $R_{2a}$ für Chlor, Brom oder Methyl steht, werden Verbindungen der Formel Ia in 5-Stellung methyliert oder halogeniert.

Die Oxydation der Verbindungen der Formel II sowie die unter a), b) und c) erwähnten Verfahren können nach an sich bekannten Methoden erfolgen. Diese Methoden sind insbesondere aus der Chemie der Mutterkornalkaloide bekannt, da die neuen Verbindungen die Struktur eines Ergolins, das in 8,9-Stellung mit einem Phenylring anneliert ist, aufweisen.

Die Oxydation der Verbindungen der Formel II wird z. B. mit $MnO_2$ als mildem Oxydationsmittel durchgeführt.

Die unter a) beschriebene Desalkylierung wird besonders mittels hydrogenolytischer Debenzylierung oder mittels Demethylierung mit BrCN und $K_2CO_3$ in $CHCl_3$ und nachfolgender Reduzierung mit Essigsäure und Zn in Wasser ausgeführt.

Die unter b) beschriebene Alkylierung kann in bekannter Weise selektiv, das heisst ohne dass zusätzlich das 7-N-Atom alkyliert wird, ausgeführt werden, indem Gebrauch gemacht wird vom stärker aziden Charakter des H-Atoms an $N_4$. Man setzt dazu die Ausgangsverbindung der Formel I'' mit einem Äquivalent einer starken Base, besonders NaH um, wodurch das Mono-Na-Salz dieses Ausgangsproduktes (Salzbildung nur am 4-N-Atom) gebildet wird. Das Salz kann nun leicht mit der äquivalenten Menge eines Alkylhalogenids umgesetzt werden, wodurch selektiv eine Alkylierung des 4-N-Atoms stattfindet. Für die Methylierung kann z. B. Methyliodid verwendet werden.

Die unter c) beschriebene Halogenierung kann z. B. für die Einführung eines Bromatoms mit N-Bromsuccinimid und für die Einführung eines Chloratoms mit $SO_2Cl_2$ und Bortrifluorid-etherat erfolgen.

Für die Methylierung wird z. B. das Ausgangsprodukt der Formel Ia mit Dithiolan und $SO_2Cl_2$ umgesetzt und nachfolgend mit Raney-Ni entschwefelt.

Die Ausgangsprodukte, welche für die oben beschriebenen Verfahren eingesetzt werden, bestehen aus verschiedenen 6a,12b-trans-Stereoisomeren. Jedes der oben beschriebenen Verfahren kann mit Ausgangsprodukten in Form der individuellen optisch aktiven Isomeren oder deren Isomergemische, besonders deren Racemate ausgeführt werden und führt dann zu den entsprechenden Endprodukten.

Die Racemate können in die individuellen optisch aktiven Komponenten gespalten werden, wobei bekannte Methoden zur Anwendung gelangen, z. B. via vorübergehende Säureadditionssalzbildung mit optisch aktiven Säuren, z. B. (+)- oder (-)-Mandelsäure, und fraktionierter Kristallisation der diastereoisomeren Säureadditionssalze.

Die neuen Verbindungen können nach bekannten Methoden in freier Form oder in Salzform, z. B. Säureadditionssalzform isoliert werden.

Die Ausgangsverbindungen der Formel II können hergestellt werden, indem man Verbindungen der Formel III,

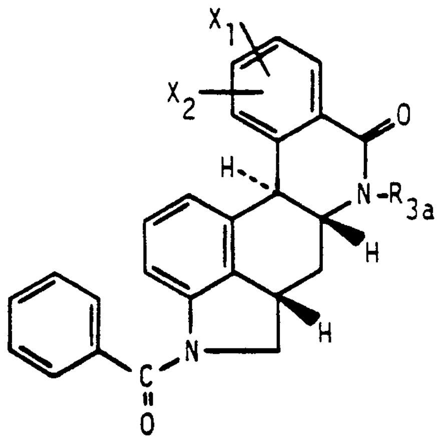

III

worin

$R_{3a}$, $X_1$ und $X_2$ obige Bedeutung besitzen, nach an sich bekannten Methoden, z. B. mittels $LiAlH_4$ oder $B_2H_6$ reduziert und gegebenenfalls in 4-Stellung alkyliert.

Zu den Verbindungen der Formel III gelangt man, indem man Verbindungen der Formel IV,

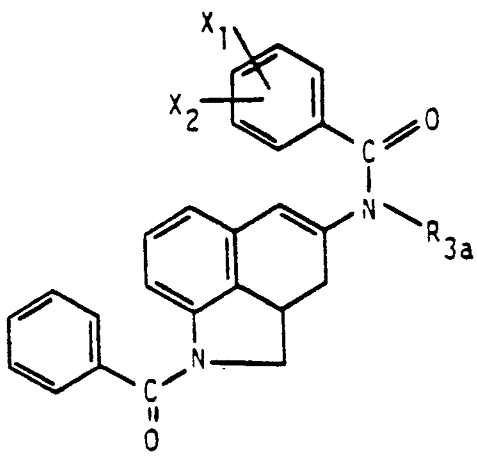

IV

worin

$R_{3a}$, $X_1$ und $X_2$ obige Bedeutung besitzen, photocyclisiert.

Die Verbindungen der Formel IV können erhalten werden, indem man die Verbindung der Formel V

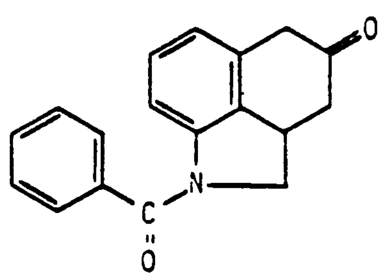

V

mit Verbindungen der Formel VI,

$H_2N\text{-}R_{3a}$ VI

worin

$R_{3a}$ obige Bedeutung besitzt, und anschliessend mit Verbindungen der Formel VII,

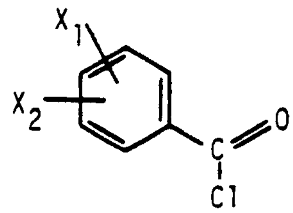

VII

worin

$X_1$ und $X_2$ obige Bedeutung besitzen, nach an sich bekannten Methoden umsetzt.

Die Verbindungen der Formeln V, VI und VII sind bekannt oder nach an sich bekannten Verfahren herstellbar.

Die Verbindungen der Formel I und ihre physiologisch verträglichen Säureadditionssalze, im folgenden als erfindungsgemässe Verbindungen bezeichnet, weisen im Tierversuch interessante pharmakologische Eigenschaften auf und können daher als Heilmittel verwendet werden.

Sie zeigen eine Dopaminrezeptor-stimulierende Aktivität am kardiovaskulären System, was nachgewiesen wird, indem sie eine Blutdruckerniedrigung und einen verminderten Gefässwiderstand der oberen Gekröseschlagader im narkotisierten Hund auslösen [B.J. Clark, Postgrad. Med. J. 57 (Suppl. 1) 45 - 54].

Für diesen Versuch werden Hunde verwendet, welche mit Chloralose und Urethan narkotisiert werden. Der Blutdruck wird mittels eines Katheters in der Oberschenkelschlagader und der Blutzufuhr in der oberen Gekröseschlagader mittels elektromagnetischer Durchblutungssonde gemessen.

Die erfindungsgemässen Verbindungen verursachen eine Blutdruckerniedrigung und eine Verminderung des Widerstandes in den Gekrösearterien bei einer Dosis zwischen 100 und 10000 µg/KG.

Die erfindungsgemässen Verbindungen sind daher brauchbar als Dopaminrezeptorstimulatoren, z. B. sowohl zur Behandlung von kongestiver Herzinsuffizienz als auch von Hypertonie oder von Nierenversagen mit verminderter Harnausscheidung.

Eine geeignete tägliche Dosis liegt zwischen etwa 200 bis etwa 1000 mg; zweckmässig enthalten Dosierungsformen, welche für orale Verabreichung geeignet sind, etwa 50 bis 500 mg der erfindungsgemässen Verbindungen in freier oder in pharmazeutisch annehmbarer Salzform, mit einem pharmazeutisch annehmbaren Verdünnungsmittel oder Träger vermischt.

Die erfindungsgemässen Verbindungen zeigen besonders eine dopaminerge Aktivität in vivo am zentralen Nervensystem, was nachgewiesen wird durch eine kontralaterale Rotation beim Verabreichen in Dosen von 1 bis 10 mg/kg p.o. oder 0,3 mg/kg s.c. an Ratten, bei denen durch eine 6-Hydroxydopamin-Injektion eine unilaterale Verletzung der nigroneostriatalen Dopaminbahn verursacht wurde [U. Ungerstedt, Acta physiol. scand. Suppl. 367, 69 - 93 (1973)].

Die erfindungsgemässen Verbindungen zeigen ebenfalls Stereotypie im Apomorphin-Stereotypieversuch bei Verabreichung in Mengen von etwa 30 mg/kg p.o.

In vitro stimulieren die erfindungsgemässen Verbindungen dopaminsensitive Adenylatcyclase im Striatumhomogenat der Ratte, hemmen jedoch nicht die elektrisch hervorgerufene Freisetzung von Acetylcholin aus Striatumscheibchen der Ratte (Versuche gemäss Eur. J. Pharmacol. 95, 101).

Die erfindungsgemässen Verbindungen sind besonders brauchbar als selektive dopaminerge Wirkstoffe, z. B. zur Behandlung von Morbus Parkinson.

Eine geeignete tägliche Dosis liegt zwischen etwa 1 bis etwa 100 mg und zweckmässig enthalten Dosierungsformen, welche für orale Verabreichung geeignet sind, etwa 0,25 bis etwa 50 mg der erfindungsgemässen Verbindungen in freier oder in pharmazeutisch annehmbarer Salzform, mit einem pharmazeutisch annehmbaren Verdünnungsmittel oder Träger vermischt.

Die erfindungsgemässen Verbindungen zeichnen sich überdies durch eine analgetische Wirkung aus. Diese zeigt sich beispielsweise im Hot plate-Test an der Maus [Methode nach Eddy und Leimbach, J. Pharmac. Exp. Ther. 107, 385 (1953)] bei Dosen von 10 bis 100 mg/kg Körpergewicht p.o., im Arthritis-Schmerz-Test [basierend auf dem von A.W. Pircio et al. in Eur. J. Pharmacol. 31, 207 - 215 (1975) beschriebenen Test] an der Ratte bei Dosen von 5 bis 50 mg/kg Körpergewicht p.o. sowie im Step-Reiz-Test beim Rhesus Affen [basierend auf den von J.J. Boren et al. in Amer. J. Physiol. 201, 429 (1961), E.D. Weitzman et al. in Neurology 12, 264 (1962) und B. Weiss et al. in J. Pharmacol. Exp. Ther. 131, 120 (1961) und 143, 169 (1964) beschriebenen Methoden] bei Dosen von 5 bis 50 mg/kg Körpergewicht p.o.

Aufgrund ihrer analgetischen Wirkung können die Substanzen zur Behandlung von Schmerzen verschiedener Genese verwendet werden.

Eine geeignete Tagesdosis liegt zwischen etwa 50 bis 150 mg. So enthalten z. B. für orale Applikation die Teildosen etwa 10 bis 75 mg der erfindungsgemässen Verbindungen neben festen und flüssigen Trägersubstanzen.

Folglich betrifft die Erfindung die Anwendung der erfindungsgemässen Verbindungen als Pharmazeutika, beispielsweise bei der therapeutischen Behandlung, z. B. zur Behandlung der Parkinson'schen Krankheit, von Herzkreislauferkrankungen oder von Ausfallerscheinungen des Zentralnervensystems, oder als Analgetikum.

Als Heilmittel können die erfindungsgemässen Verbindungen allein oder in geeigneter Arzneiform mit

pharmakologisch indifferenten Stoffen verabreicht werden.

Gegenstand der Erfindung sind auch pharmazeutische Zusammensetzungen, die die erfindungsgemässen Verbindungen enthalten. Für ihre Herstellung können die in der Pharmazie gebräuchlichen Hilfs- und Trägerstoffe verwendet werden. Geeignete galenische Formen sind z. B. Tabletten und Kapseln.

In den nachfolgenden Beispielen, die die Erfindung näher erläutern, erfolgen alle Temperaturangaben in Celsiusgraden und sind unkorrigiert.

**Beispiel 1:**

**trans-4,6,6a,7,8,12b-Hexahydro-7-methylindolo[4,3-ab]phenanthridin**

a) <u>1,N-Dibenzoyl-4-methylamino-1,2,2a,3-tetrahydrobenz[c,d]indol</u>

30 g 1-Benzoyl-1,2,2a,3,4,5-hexahydro-4-ketobenz[c,d]indol werden zusammen mit 1,5 g eines sauren Montmorillonit-Katalysators in 1200 ml Diäthyläther suspendiert. In die gerührte Suspension leitet man bei Zimmertemperatur Methylamin ein, entweichendes Methylamin wird an einem mit Trockeneis beschickten Kühler rekondensiert und in die Reaktionslösung zurückgeleitet. Im Laufe der Reaktion findet eine Umfällung statt, der Reaktionsverlauf wird dabei dünnschichtchromatographisch verfolgt. Nach Beendigung der Reaktion wird überschüssiges Methylamin mittels Durchblasen von Stickstoff aus der Reaktionslösung entfernt, der entstandene Niederschlag, das entstandene rohe Enamin, abfiltriert, in 600 ml Methylenchlorid gelöst und klarfiltriert. Das Filtrat wird auf 0 abgekühlt und mit 14,6 ml Triäthylamin versetzt. Zu dieser Lösung tropft man nun unter Rühren bei 0 ° 12,4 ml Benzoylchlorid. Nach beendeter Zugabe entfernt man die Kühlung und lässt noch während 14 Stunden bei Zimmertemperatur weiterrühren. Die Reaktionslösung wird anschliessend mit 2n aq Salzsäure, gefolgt von 2n Ammoniaklösung extrahiert, die vereinigten organischen Phasen mit Salzlösung gewaschen, über Magnesiumsulfat getrocknet, filtriert und eingeengt. Man erhält das rohe Produkt als gelbes Öl, das in dieser Form weiterverarbeitet wird.

b) <u>(5aR*,6aS*,12bS*)-4-Benzoyl-4,5,5a,6,6a,7,8,12b-octahydro-8-keto-7-methylindolo[4,3-ab]phenanthridin</u>

40 g 1,N-Dibenzoyl-4-methylamino-1,2,2a,3-tetrahydrobenz[c,d]indol werden in 2 Liter Aceton gelöst und mit einem Hochdruckbrenner (250 Watt) mit Quarzfinger bestrahlt. Es entsteht ein fester Niederschlag, der erstmals nach 4 Stunden abfiltriert wird. Die Bestrahlung wird fortgesetzt bis dünnschichtchromatographisch kein Edukt mehr in der Reaktionslösung nachgewiesen werden kann. Der Niederschlag wird in Methylenchlorid/Methanol = 1/1 gelöst, mit Aktivkohle gekocht, über Kieselgur filtriert und bis zur beginnenden Kristallisation eingeengt. Das abfiltrierte, gewaschene, getrocknete kristalline Produkt weist einen Schmelzpunkt von 292 - 295° auf.

c) <u>(5aR*,6aS*,12bS*)-4,5,5a,6,6a,7,8,12b-Octahydro-7-methylindolo[4,3-a,b]phenanthridin</u>

8,5 g Lithiumaluminiumhydrid werden unter Stickstoff in 500 ml Diäthylether suspendiert. Zu dieser Suspension tropft man unter Rühren eine Lösung von 17,1 g (5aR*,6aS*,12bS*)-4-Benzoyl-4,5,5a,6,6a,7,8,12b-octahydro-8-keto-7-methylindolo[4,3-a,b]phenanthridin in 150 ml Tetrahydrofuran. Die Temperatur der Reaktionslösung steigt dabei bis zum Siedepunkt an. Die Reaktionslösung wird noch 2 Stunden nachgerührt und anschliessend durch Zugabe von konzentrierter wässriger Natriumhydroxyd-Lösung hydrolysiert, mit Magnesiumsulfat und Kieselgur versetzt und filtriert. Der Filterkuchen wird in 2n wässrigem Ammoniumhydroxyd suspendiert und mit Essigester nachextrahiert. Die vereinigten organischen Phasen werden über Magnesiumsulfat getrocknet, filtriert und eingeengt. Das so erhaltene Öl wird aus Essigsäureäthylester kristallisiert. Nach Abfiltrieren, Waschen und Trocknen erhält man das kristalline Produkt mit einem Schmelzpunkt von 149 - 150°.

d) <u>trans-4,6,6a,7,8,12b-Hexahydro-7-methylindolo[4,3-ab]phenantridin</u>

2 g (5aR*,6aS*,12bS*)-4,5,5a,6,6a,7,8,12b-Octahydro-7-methylindolo[4,3-ab]phenanthridin werden in 250 ml Methylenchlorid gelöst. Anschliessend werden 20 g aktiv gefälltes Mangandioxid in die Lösung suspendiert und die erhaltene Suspension bei Zimmertemperatur gerührt, bis ein Dünnschichtchromatogramm kein Edukt mehr anzeigt. Die Reaktionslösung wird nun über Kieselgur filtriert, eingeengt und das erhaltene Öl aus Methylenchlorid kristallisiert. Man erhält die kristalline Titelverbindung, die bei 215° schmilzt.

Analog Beispiel 1 werden folgende Verbindungen der Formel Ia hergestellt (alles Racemate):

| Beispiel | $R_1$ | $X_1$ | $X_2$ | $R_{3a}$ | Smp. |
|---|---|---|---|---|---|
| 1a | H | H | H | $-CH_2-CH_3$ | 227 - 229° (2) |
| 1b | H | H | H | $-CH_2-CH_2-CH_3$ | 224 - 225° (3) |
| 1c | $-CH_3$ | H | H | $-CH_3$ | 184 - 185° (1) |

| | | | | | |
|---|---|---|---|---|---|
| 1d | H | H | H | $-CH_2-CH=CH_2$ | 203° (1) |
| 1e | H | H | H | $-CH_2-$ 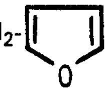 | 230° (1) |
| 1f | H | H | H | $-CH_2-$ 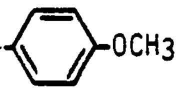 | 213° (1) |
| 1g | H | 11-$OCH_3$ | H | $-CH_3$ | 211° (1) |
| 1h | H | 11-OH | H | $-CH_3$ | 207° (1) |
| 1i | H | 10-$OCH_3$ | H | $-CH_3$ | 225° (1) |
| 1j | H | 10-OH | H | $-CH_3$ | 245° (1) |
| 1k | H | 10-$CH_3$ | H | $-CH_3$ | 211° (1) |
| 1l | H | 11-$CH_3$ | H | $-CH_3$ | 212° (1) |
| 1m | H | 10-$CF_3$ | H | $-CH_3$ | 230° (1) |
| 1n | H | 10-$CH_3$ | 12-$CH_3$ | $-CH_3$ | 191° (1) |
| 1o | H | 11-Cl | H | $-CH_3$ | 179° (1) |
| 1p | H | 12-OH | H | $-CH_3$ | 265° (1) |

(1) Base
(2) Hydrogenfumarat
(3) Fumarat

**Beispiel 2:**

**(-)-trans-4,6,6a,7,8,12b-Hexahydro-7-methylindolo[4,3-ab]phenanthridin**

a) (+)-(5aR*,6aS*,12bS*)-4,5,5a,6,6a,7,8,12b-Octahydro-7-methylindolo[4,3-ab]phenanthridin

50 g (±)-(5aR*,6aS*,12bS*)-4,5,5a,6,6a,7,8,12b-Octahydro-7-methylindolo[4,3-ab]phenanthridin werden zusammen mit 27,5 g (+)-Mandelsäure bei 70° in 500 ml Äthanol gelöst. Die Lösung wird über Nacht bei Zimmertemperatur stehen gelassen und das entstandene Kristallisat wird anschliessend abfiltriert, mit Äthanol/Äther = 1/1, gefolgt von Äther, gewaschen und getrocknet. Das so erhaltene Kristallisat (Smp. 184 - 185°) wird in 400 ml Äthanol heiss gelöst und über Nacht zur Kristallisation stehen gelassen.
Man erhält ein zweites Kristallisat, das abfiltriert und entsprechend dem ersten Kristallisat gewaschen und getrocknet wird. Das zweite Kristallisat wird nun durch Extraktion mit 1n wässriger Natriumkarbonat-Lösung/Methylenchlorid in die freie Base überführt, die eine optische Drehung von $[\alpha]^{20}_D$ = + 378,0° (c = 1,0/DMF) aufweist und in dieser Form weiterverarbeitet wird.

b) (-)-trans-4,6,6a,7,8,12b-Hexahydro-7-methylindolo[4,3-ab]phenanthridin

(-)-trans-4,6,6a,7,8,12b-Hexahydro-7-methylindolo[4,3-ab]phenanthridin erhält man auf die in Beispiel 1 beschriebene Weise aus (+)-(5aR*,6aS*,12bS*)-4,5,5a,6,6a,7,8,12b-Octahydro-7-methylindolo[4,3-ab]-phenanthridin. Die aus Methylenchlorid/Methanol = 1/1 kristallisierte Verbindung schmilzt bei 215°, $[\alpha]^{20}_D$ = -610,7° (c = 0,84/DMF).

**Beispiel 3:**

**(+)-trans-4,6,6a,7,8,12b-Hexahydro-7-methylindolo[4,3-ab]phenanthridin**

a) (-)-(5aR*,6aS*,12bS*)-4,5,5a,6,6a,7,8,12b-Octahydro-7-methylindolo[4,3-ab]phenanthridin

Aus dem gleichen Ausgangsmaterial wie unter Beispiel 2 a) erhält man unter Verwendung der (-)-Mandelsäure analog die entsprechende (-)-drehende Form.

b) (+)-trans-4,6,6a,7,8,12b-Hexahydro-7-methylindolo[4,3-ab]phenanthridin

(+)-trans-4,6,6a,7,8,12b-Hexahydro-7-methylindolo-[4,3-ab]phenanthridin erhält man entsprechend aus (-)-(5aR*,6aS*,12bS*)-4,5,5a,6,6a,7,8,12b-Octahydro-7-methylindolo[4,3-ab]phenanthridin, $[\alpha]^{20}_D$ = + 613,1° (c = 0,83/DMF).

**Beispiel 4:**

**trans-4,6,6a,7,8,12b-Hexahydroindolo[4,3-ab]phenanthridin**

38,6 g Bromcyan werden in 400 ml Chloroform gelöst. Zur Lösung gibt man unter Rühren 20 g trans-4,6,6a,7,8,12b-Hexahydro-7-methylindolo[4,3-ab]phenanthridin, gefolgt von 10 g Kaliumkarbonat.

Die entstandene Suspension wird über Nacht bei Zimmertemperatur gerührt, anschliessend filtriert und das Filtrat eingeengt. Der Eindampfrückstand wird unter Erwärmen in 400 ml konzentrierter Essigsäure gelöst und 55 ml Wasser, gefolgt von 47,5 g Zinkpulver beigefügt. Die entstandene Suspension wird nun während 18 Stunden bei 90° gerührt. Die Reaktionslösung wird anschliessend filtriert, der Filterrückstand mit Methylenchlorid/Methanol nachgewaschen und die vereinigten Filtrate eingedampft. Der Eindampfrückstand wird in Methylenchlorid/Methanol = 9/1 aufgenommen und mit 1n wässriger Natriumkarbonatlösung extrahiert. Die vereinigten organischen Phasen werden mit Kochsalzlösung gewaschen, über Magnesiumsulfat getrocknet, filtriert, eingeengt und der Rückstand an Kieselgel mittels Methylenchlorid/Methanol = 95/5 ($CH_2Cl_2$ 10 %-ig gesättigt mit $NH_3$) chromatographiert. Man erhält die Titelverbindung, deren Hydrochlorid, kristallisiert aus Methanol, bei 301 - 303° schmilzt.

Analog Beispiel 4 werden folgende Verbindungen der Formel Ib hergestellt:

| Beispiel | $R_2$ | $X_1$ | $X_2$ | Racemat/ Antipode | Smp. |
|---|---|---|---|---|---|
| 4a | H | 11-$OCH_3$ | H | Racemat | 283° (1) |
| 4b | H | H | H | (-)* | 311 - 313° (2) |

(1) Base
(2) Hydrochlorid
* $[\alpha]^{20}_D$ = - 436,8° (c = 0,7/DMF)

**Beispiel 5:**

**trans-4,6,6a,7,8,12b-Hexahydro-4,7-dimethylindolo[4,3-ab]phenanthridin**

3 g trans-4,6,6a,7,8,12b-Hexahydro-7-methylindolo[4,3-ab]phenanthridin werden in 60 ml Dimethylformamid gelöst. Die Lösung wird auf - 25° abgekühlt und unter Rühren mit 280 mg einer 55-%-igen Natriumhydrid-Dispersion versetzt. Man lässt die entstandene Suspension während 40 Minuten auf 0° aufwärmen und tropft dann 0,81 ml Methyljodid, gelöst in 10 ml Dimethylformamid zu. Die schwarze Reaktionslösung wird noch 1 Stunde bei 0° weitergerührt und anschliessend am Hochvakuum vom Dimethylformamid befreit. Der Eindampfrückstand wird in Methylenchlorid/Methanol = 95/5 aufgenommen, mit Wasser extrahiert und die vereinigten organischen Phasen getrocknet, filtriert und eingeengt. Der Eindampfrückstand wird an Kieselgel mit Methylenchlorid/Methanol = 98/2 (Methylenchlorid 10-%-ig gesättigt mit $NH_3$) chromatographiert. Man erhält die Titelverbindung, die aus dem zur Chromatographie verwendeten Lösungsgemisch kristallisiert und nach Waschen mit Äther und anschliessendem Trocknen bei 184 - 185° schmilzt.

**Beispiel 6:**

**trans-5-Bromo-4,6,6a,7,8,12b-hexahydro-7-methylindolo[4,3-ab]phenanthridin**

4 g trans-4,6,6a,7,8,12b-Hexahydro-7-methylindolo[4,3-ab]phenanthridin werden in 300 ml Chloroform suspendiert und die gerührte Suspension auf Rückflusstemperatur erhitzt. Man versetzt jetzt rasch mit einer Lösung von 2,7 g Bromsuccinimid in 100 ml Chloroform und erhitzt die nun klare Reaktionslösung während einer weiteren Stunde am Rückfluss. Die abgekühlte Reaktionslösung wird mit in wässriger Natriumkarbonatlösung extrahiert, die organischen Phasen getrocknet, eingeengt und der Eindampfrückstand an Kieselgel mit Methylenchlorid/Methanol = 95/5 (Methylenchlorid 10 %-ig gesättigt mit $NH_3$) chromatographiert. Man erhält die Titelverbindung, deren Fumarat aus Methanol kristallisiert (Smp. 235 - 237°).

Entsprechend den für die Herstellung 2-substituierter Ergolinderivate beschriebenen Verfahren werden auch folgende Verbindungen der Formel Id aus den entsprechenden Verbindungen der Formel Ia hergestellt:

| Beispiel | $R_1$ | $R_{2a}$ | $R_{3a}$ | $X_1$ | $X_2$ | Racemat/Antipode | Smp. |
|---|---|---|---|---|---|---|---|
| 6a | H | Cl | $-CH_3$ | H | H | Racemat | 239 - 240° (2) |
| 6b | H | $-CH_3$ | $-CH_3$ | H | H | Racemat | 201 - 204° (1) |
| 6c | H | BR | $-CH_3$ | H | H | (-)* | 235 - 237° (1) |
| 6d | H | $-CH_3$ | $-CH_3$ | H | H | (-)** | 226 - 229° (1) |

(1) Base
(2) Fumarat
* $[\alpha]^{20}_D = -550{,}8°$ (c = 1,1/DMF)
** $[\alpha]^{20}_D = -587{,}8°$ (c = 0,64/DMF)

**Patentansprüche** für die Vertragsstaaten: BE, CH, DE, FR, GB, IT, LI, LU, NL, SE

1. Eine Verbindung der Formel I,

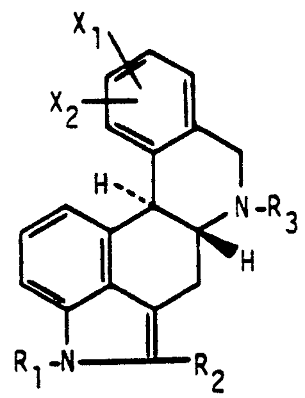

I

worin
$R_1$ und $R_3$ unabhängig voneinander Wasserstoff, $(C_{1-4})$Alkyl, $(C_{3-5})$Alkenyl, wobei die Doppelbindung nicht zu dem Stickstoffatom benachbart ist, $(C_{3-6})$Cycloalkyl$(C_{1-3})$alkyl, im Phenylring gegebenenfalls durch $(C_{1-4})$Alkyl, $(C_{1-4})$Alkoxy, Hydroxy oder Halogen substituiertes Phenyl$(C_{1-3})$alkyl oder 2- oder 3-Furyl$(C_{1-3})$alkyl bedeuten,
$R_2$ für Wasserstoff, Chlor, Brom oder Methyl steht,
und
$X_1$ und $X_2$ unabhängig voneinander für Wasserstoff, $(C_{1-4})$Alkyl, $(C_{1-4})$Alkoxy, Hydroxy, Halogen oder Trifluormethyl stehen,
in Form der freien Base oder eines Säureadditionssalzes.

2. Eine Verbindung der Formel I gemäss Anspruch 1,
worin
$R_1$ und $R_3$ unabhängig voneinander Wasserstoff, $(C_{1-4})$Alkyl, $(C_{3-5})$Alkenyl, wobei die Doppelbindung nicht zu dem Stickstoffatom benachbart ist, $(C_{3-6})$Cycloalkyl$(C_{1-3})$alkyl, im Phenylring gegebenenfalls durch Methyl, Methoxy, Hydroxy oder Halogen monosubstituiertes Phenyl$(C_{1-3})$alkyl oder 2- oder 3-Furyl$(C_{1-3})$alkyl bedeuten,
$R_2$ für Wasserstoff, Chlor, Brom oder Methyl steht,
und
$X_1$ und $X_2$ unabhängig voneinander für Wasserstoff, $(C_{1-4})$Alkyl, $(C_{1-4})$Alkoxy, Hydroxy, Halogen oder Trifluormethyl stehen,
in Form der freien Base oder eines Säureadditionssalzes.

3. Eine Verbindung der Formel I gemäss Anspruch 1,
worin
$R_1$ Wasserstoff oder $(C_{1-4})$Alkyl bedeutet,
$R_2$ für Wasserstoff, Chlor, Brom oder Methyl steht,
$R_3$ Wasserstoff, $(C_{1-4})$Alkyl, gegebenfalls durch $(C_{1-4})$Alkoxy monosubstituiertes Benzyl oder 2-Furylmethyl bedeutet,
$X_1$ in 10-, 11- oder 12-Stellung steht und Wasserstoff, $(C_{1-4})$Alkyl, $(C_{1-4})$Alkoxy, Hydroxy, Halogen oder Trifluormethyl bedeutet und
$X_2$ für Wasserstoff steht,
in Form der freien Base oder eines Säureadditionssalzes.

4. Eine Verbindung der Formel I gemäss Anspruch 1 aus der Gruppe der Verbindungen der Formel I in racemischer Form oder mit der angegebenen Konfiguration, worin $R_1$, $R_2$, $X_1$, $X_2$, beziehungsweise $R_3$, folgende Bedeutungen haben:

H, H, H, H, Methyl
H, H, H, H, Ethyl
H, H, H, H, n-Propyl
Methyl, H, H, H, Methyl
H, H, H, H, Allyl
H, H, H, H, 2-Furylmethyl
H, H, H, H, p-Methoxybenzyl
H, H, 11-Methoxy, H, Methyl
H, H, 11-Hydroxy, H, Methyl
H, H, 10-Methoxy, H, Methyl
H, H, 10-Hydroxy, H, Methyl
H, H, 10-Methyl, H, Methyl
H, H, 11-Methyl, H, Methyl
H, H, 10-Trifluormethyl, H, Methyl
H, H, 10-Methyl, 12-Methyl, Methyl
H, H, 11-Chlor, H, Methyl
H, H, 12-Hydroxy, H, Methyl
H, H, H, H, Methyl (+)
H, H, H, H, H
H, H, 11-Methoxy, H, H
H, H, H, H, H (-)
Methyl, H, H, H, Methyl
H, Brom, H, H, Methyl
H, Chlor, H, H, Methyl
H, Methyl, H, H, Methyl
H, Brom, H, H, Methyl (-)
H, Methyl, H, H, Methyl (-)
in Form der freien Base oder eines Säureadditionssalzes.

5. Das (-)-trans-4,6,6a,7,8,12b-Hexahydro-7-methylindolo[4,3-ab]phenanthridin in Form der freien Base oder eines Säureadditionssalzes.

6. Verfahren zur Herstellung der Verbindungen der Formel gemäss Anspruch 1 und ihrer Säureadditionssalze, dadurch gekennzeichnet, dass man Verbindungen der Formel II,

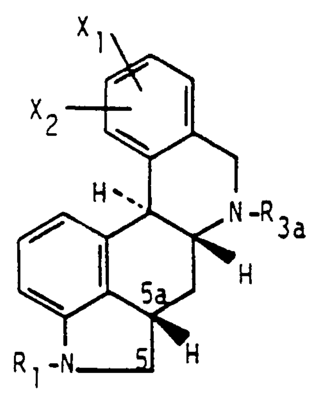
II

worin
$R_1$, $X_1$ und $X_2$ wie im Anspruch 1 definiert sind und $R_{3a}$ die im Anspruch 1 für $R_3$ angegebene Bedeutung mit Ausnahme von Wasserstoff besitzt, in 5,5a-Stellung des Indolophenanthridingerüstes oxydiert, die erhaltenen Verbindungen der Formel Ia,

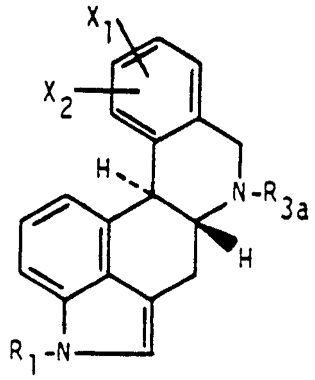

Ia

worin

$R_1$, $R_{3a}$, $X_1$ und $X_2$ obige Bedeutung besitzen gewünschtenfalls in die übrigen Verbindungen der Formel I überführt und die erhaltenen Verbindungen der Formel I als freie Basen oder als Säureadditionssalze gewinnt.

7. Verfahren zur Verstellung von Verbindungen der Formel Ib,

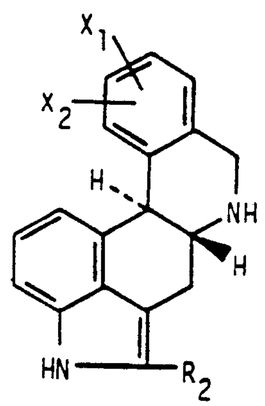

Ib

worin

$R_2$, $X_1$ und $X_2$ wie im Anspruch 1 definiert sind, und ihren Säureadditionssalzen, dadurch gekennzeichnet, dass man Verbindungen der Formel I',

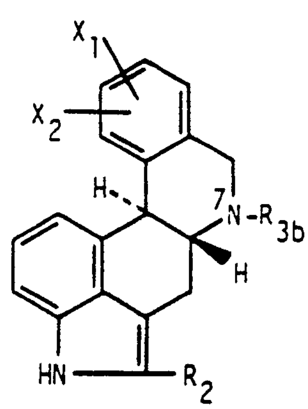

I'

worin

$R_2$, $X_1$ und $X_2$ wie im Anspruch 1 definiert sind und $R_{3b}$ Methyl oder Benzyl bedeutet, in 7-Stellung desalkyliert und die erhaltenen Verbindungen der Formel Ib als freie Basen oder als Säureadditionssalze gewinnt.

8. Verfahren zur Herstellung von Verbindungen der Formel Ic,

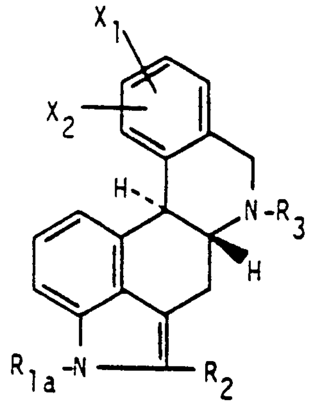

Ic

worin

$R_2$, $R_3$, $X_1$ und $X_2$ wie im Anspruch 1 definiert sind und $R_{1a}$ die im Anspruch 1 für $R_1$ angegebene Bedeutung mit Ausnahme von Wasserstoff besitzt, und ihren Säureadditionssalzen, dadurch gekennzeichnet, dass man Verbindungen der Formel I',

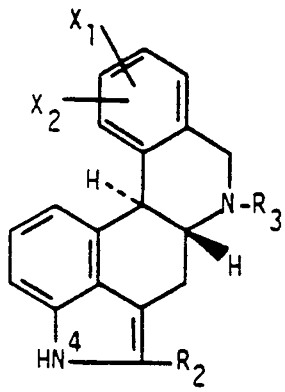

I"

worin

$R_2$, $R_3$, $X_1$ und $X_2$ wie im Anspruch 1 definiert sind, in 4-Stellung alkyliert und die erhaltenen Verbindungen der Formel Ic als freie Basen oder als Säureadditionssalze gewinnt.

9. Verfahren zur Herstellung von Verbindungen der Formel Id,

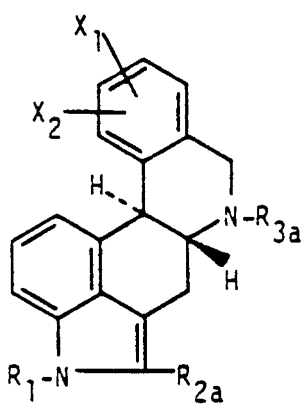

Id

worin

$R_1$ $R_{3a}$, $X_1$ und $X_2$ wie in den Ansprüchen 1 und 6 definiert sind und $R_{2a}$ für Chlor, Brom oder Methyl steht, und ihren Säureadditionssalzen, dadurch gekennzeichnet, dass man Verbindungen der Formel Ia gemäss Anspruch 6 in 5-Stellung methyliert oder halogeniert und die erhaltenen Verbindungen der Formel Id als freie Basen oder als Säureadditionssalze gewinnt.

10. Eine Verbindung gemäss einem der Ansprüche 1 bis 5, in freier oder pharmazeutisch annehmbarer Säureadditionssalzform, zur Anwendung als Pharmazeutikum.

11. Eine Verbindung gemäss einem der Ansprüche 1 bis 5, in freier oder pharmazeutisch annehmbarer Säureadditionssalzform, zur Behandlung der Parkinson'schen Krankheit, von Herzkreislauferkrankungen oder

von Ausfallerscheinungen des Zentralnervensystems, oder zur Anwendung als Analgetikum.

12. Eine pharmazeutische Zusammensetzung, enthaltend eine Verbindung gemäss einem der Ansprüche 1 bis 5, in freier oder pharmazeutisch annehmbarer Säureadditionssalzform.

13. Eine Verbindung der Formel II gemäss Anspruch 6.

**Patentansprüche** für den Vertragsstaat: AT

1. Verfahren zur Herstellung von Verbindungen der Formel I,

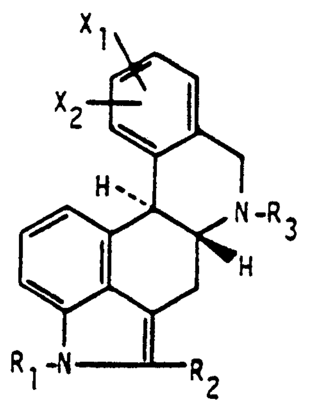

I

worin

$R_1$ und $R_3$ unabhängig voneinander Wasserstoff, $(C_{1-4})$Alkyl, $(C_{3-5})$Alkenyl, wobei die Doppelbindung nicht zu dem Stickstoffatom benachbart ist, $(C_{3-6})$Cycloalkyl$(C_{1-3})$alkyl, im Phenylring gegebenenfalls durch $(C_{1-4})$Alkyl, $(C_{1-4})$Alkoxy, Hydroxy oder Halogen substituiertes Phenyl$(C_{1-3})$alkyl oder 2- oder 3-Furyl$(C_{1-3})$alkyl bedeuten,

$R_2$ für Wasserstoff, Chlor, Brom oder Methyl steht,

und

$X_1$ und $X_2$ unabhängig voneinander für Wasserstoff, $(C_{1-4})$Alkyl, $(C_{1-4})$Alkoxy, Hydroxy, Halogen oder Trifluormethyl stehen,

in Form der freien Basen oder von Säureadditionssalzen, dadurch gekennzeichnet, dass man Verbindungen der Formel II,

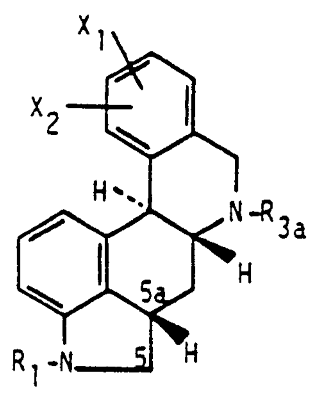

II

worin

$R_1$, $X_1$ und $X_2$ obige Bedeutung besitzen und $R_{3a}$ die für $R_3$ angegebene Bedeutung mit Ausnahme von Wasserstoff besitzt, in 5,5a-Stellung des Indolophenanthridingerüstes oxydiert, die erhaltenen Verbindungen der Formel Ia,

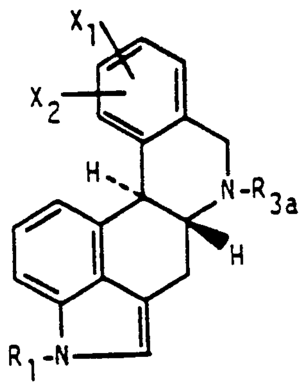

Ia

worin

$R_1$, $R_{3a}$, $X_1$ und $X_2$ obige Bedeutung besitzen, gewünschtenfalls in die übrigen Verbindungen der Formel I überführt und die erhaltenen Verbindungen der Formel I als freie Basen oder als Säureadditionssalze gewinnt.

2. Verfahren zur Herstellung von Verbindungen der Formel Ib,

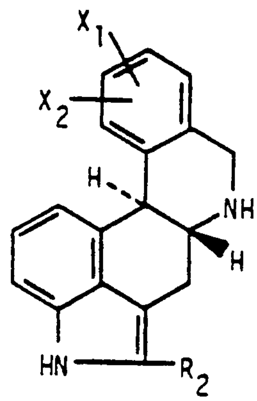

Ib

worin

$R_2$, $X_1$ und $X_2$ wie im Anspruch 1 definiert sind, und ihren Säureadditionssalzen, dadurch gekennzeichnet, dass man Verbindungen der Formel I',

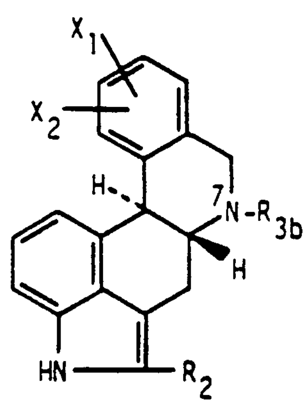

I'

worin

$R_2$, $X_1$ und $X_2$ wie im Anspruch 1 definiert sind und $R_{3b}$ Methyl oder Benzyl bedeutet, in 7-Stellung desalkyliert und die erhaltenen Verbindungen der Formel Ib als freie Basen oder als Säureadditionssalze gewinnt.

3. Verfahren zur Herstellung von Verbindungen der Formel Ic,

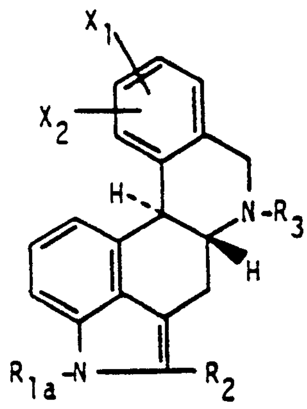

Ic

worin

$R_2$, $R_{3}$, $X_1$ und $X_2$ wie im Anspruch 1 definiert sind und $R_{1a}$ die im Anspruch 1 für $R_1$ angegebene Bedeutung mit Ausnahme von Wasserstoff besitzt, und ihren Säureadditionssalzen, dadurch gekennzeichnet, dass man Verbindungen der Formel I'',

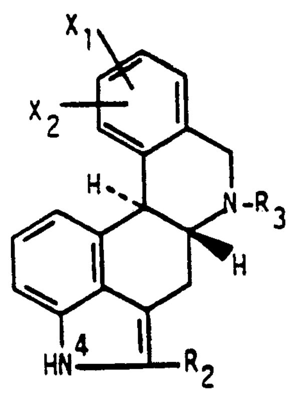

I''

worin

$R_2$, $R_3$, $X_1$ und $X_2$ wie im Anspruch 1 definiert sind, in 4-Stellung alkyliert und die erhaltenen Verbindungen der Formel Ic als freie Basen oder als Säureadditionssalze gewinnt.

4. Verfahren zur Herstellung von Verbindungen der Formel Id,

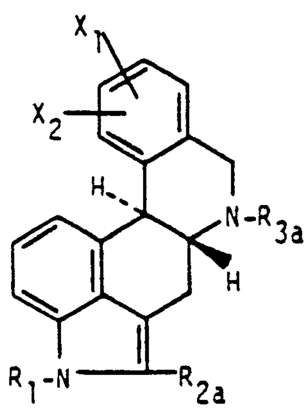

Id

worin

$R_1$, $R_{3a}$, $X_1$ und $X_2$ wie im Anspruch 1 definiert sind und $R_{2a}$ für Chlor, Brom oder Methyl steht, und ihren Säureadditionssalzen, dadurch gekennzeichnet, dass man Verbindungen der Formel Ia gemäss Anspruch 1 in 5-Stellung methyliert oder halogeniert und die erhaltenen Verbindungen der Formel Id als freie Basen oder als Säureadditionssalze gewinnt.

5. Verfahren gemäss Anspruch 1, zur Herstellung von Verbindungen der Formel I, worin

$R_1$ und $R_3$ unabhängig voneinander Wasserstoff, $(C_{1-4})$Alkyl, $(C_{3-5})$Alkenyl, wobei die Doppelbindung nicht zu dem Stickstoffatom benachbart ist, $(C_{3-6})$Cycloalkyl$(C_{1-3})$alkyl, im Phenylring gegebenenfalls durch Methyl, Methoxy, Hydroxy oder Halogen monosubstituiertes Phenyl$(C_{1-3})$alkyl oder 2- oder 3-Furyl$(C_{1-3})$alkyl bedeuten,

$R_2$ für Wasserstoff, Chlor, Brom oder Methyl steht,
und
$X_1$ und $X_2$ unabhängig voneinander für Wasserstoff, $(C_{1-4})$Alkyl, $(C_{1-4})$Alkoxy, Hydroxy, Halogen oder Trifluormethyl stehen,
in Form der freien Basen oder von Säureadditionssalzen.

6. Verfahren gemäss Anspruch 1, zur Herstellung von Verbindungen der Formel I, worin
$R_1$ Wasserstoff oder $(C_{1-4})$Alkyl bedeutet,
$R_2$ für Wasserstoff, Chlor, Brom oder Methyl steht,
$R_3$ Wasserstoff, $(C_{1-4})$Alkyl, gegebenenfalls durch $(C_{1-4})$Alkoxy monosubstituiertes Benzyl oder 2-Furylmethyl bedeutet,
$X_1$ in 10-, 11- oder 12-Stellung steht und Wasserstoff, $(C_{1-4})$Alkyl, $(C_{1-4})$Alkoxy, Hydroxy, Halogen oder Trifluormethyl bedeutet und
$X_2$ für Wasserstoff steht,
in Form der freien Basen oder von Säureadditionssalzen.

7. Verfahren gemäss Anspruch 1, zur Herstellung von Verbindungen der Formel I in racemischer Form oder mit der angegebenen Konfiguration, worin $R_1$, $R_2$, $X_1$, $X_2$, beziehungsweise $R_3$, folgende Bedeutungen haben:
H, H, H, H, Methyl
H, H, H, H, Ethyl
H, H, H, H, n-Propyl
Methyl, H, H, H, Methyl
H, H, H, H, Allyl
H, H, H, H, 2-Furylmethyl
H, H, H, H, p-Methoxybenzyl
H, H, 11-Methoxy, H, Methyl
H, H, 11-Hydroxy, H, Methyl
H, H, 10-Methoxy, H, Methyl
H, H, 10-Hydroxy, H, Methyl
H, H, 10-Methyl, H, Methyl
H, H, 11-Methyl, H, Methyl
H, H, 10-Trifluormethyl, H, Methyl
H, H, 10-Methyl, 12-Methyl, Methyl
H, H, 11-Chlor, H, Methyl
H, H, 12-Hydroxy, H, Methyl
H, H, H, H, Methyl (+)
H, H, H, H, H
H, H, 11-Methoxy, H, H
H, H, H, H, H (-)
Methyl, H, H, H, Methyl
H, Brom, H, H, Methyl
H, Chlor, H, H, Methyl
H, Methyl, H, H, Methyl
H, Brom, H, H, Methyl (-)
H, Methyl, H, H, Methyl (-)
in Form der freien Basen oder von Säureadditionssalzen.

8. Verfahren gemäss Anspruch 1, zur Herstellung des (-)-trans-4,6,6a,7,8,12b-Hexahydro-7-methyl-indolo[4,3-ab]phenanthridins, in Form der freien Base oder eines Säureadditionssalzes.

9. Ein 4,6,6a,7,8,12b-Hexahydro-indolo[4,3-ab]phenanthridin in Form der freien Base oder eines Säureadditionssalzes, hergestellt nach einem der Ansprüche 1 bis 8.

10. Eine pharmazeutische Zusammensetzung, enthaltend eine Verbindung gemäss Anspruch 9, in freier oder pharmazeutisch annehmbarer Säureadditionssalzform.

11. Ein Verfahren zur Herstellung einer pharmazeutischen Zusammensetzung, dadurch gekennzeichnet, dass man eine Verbindung der Formel I,

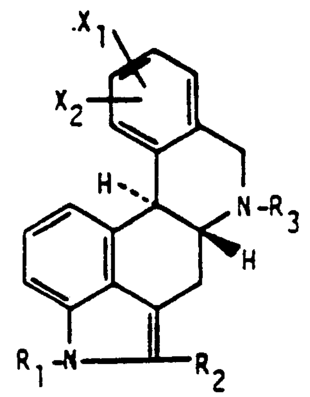

I

worin

$R_1$ und $R_3$ unabhängig voneinander Wasserstoff, $(C_{1-4})$Alkyl, $(C_{3-5})$Alkenyl, wobei die Doppelbindung nicht zu dem Stickstoffatom benachbart ist, $(C_{3-6})$Cycloalkyl$(C_{1-3})$alkyl, im Phenylring gegebenenfalls durch $(C_{1-4})$Alkyl, $(C_{1-4})$Alkoxy, Hydroxy oder Halogen substituiertes Phenyl$(C_{1-3})$alkyl oder 2- oder 3-Furyl$(C_{1-3})$alkyl bedeuten,

$R_2$ für Wasserstoff, Chlor, Brom oder Methyl steht,

und

$X_1$ und $X_2$ unabhängig voneinander für Wasserstoff, $(C_{1-4})$Alkyl, $(C_{1-4})$Alkoxy, Hydroxy, Halogen oder Trifluormethyl stehen,

in freier oder pharmazeutisch annehmbarer Säureadditionssalzform mit pharmazeutisch annehmbaren Verdünnungsmitteln oder Trägern vermischt.

12. Verfahren zur Herstellung von Verbindungen der Formel II gemäss Anspruch 1, dadurch gekennzeichnet, dass man Verbindungen der Formel III,

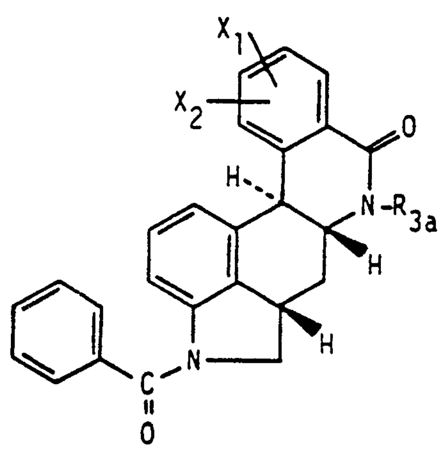

III

worin

$R_{3a}$, $X_1$ und $X_2$ wie im Anspruch 1 definiert sind, reduziert und gegebenenfalls in 4-Stellung alkyliert.

**Claims** for the Contracting States: BE, CH, DE, FR, GB, IT, LU, NL, SE

1. A compound of formula I,

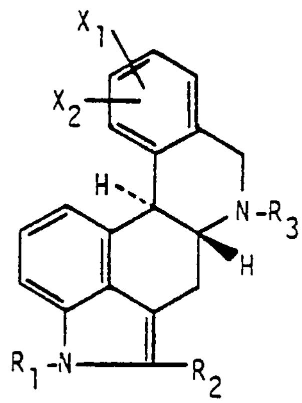

(I)

wherein

$R_1$ and $R_3$ independently are hydrogen, $(C_{1-4})$alkyl, $(C_{3-5})$alkenyl wherein the double bond is not adjacent to the nitrogen atom, $(C_{3-6})$cycloalkyl$(C_{1-3})$alkyl, phenyl$(C_{1-3})$alkyl optionally substituted in the phenyl ring by $(C_{1-4})$alkyl, $(C_{1-4})$alkoxy, hydroxy or halogen, or 2- or 3-furyl$(C_{1-3})$alkyl,

$R_2$ is hydrogen, chlorine, bromine or methyl,

$X_1$ and $X_2$ independently are hydrogen, $(C_{1-4})$alkyl, $(C_1$-4)alkoxy, hydroxy, halogen or trifluoromethyl,

in free base or acid addition salt form.

2. A compound of formula I according to claim 1, wherein

$R_1$ and $R_3$ independently are hydrogen, $(C_{1-4})$alkyl, $(C_{3-5})$alkenyl wherein the double bond is not adjacent to the nitrogen atom, $(C_{3-6})$cyclo-alkyl$(C_{1-3})$alkyl, phenyl$(C_{1-3})$alkyl optionally monosubstituted in the phenyl ring by methyl, methoxy, hydroxy or halogen, or 2- or 3-furyl$(C_{1-3})$alkyl,

$R_2$ is hydrogen, chlorine, bromine or methyl,

$X_1$ and $X_2$ independently are hydrogen, $(C_{1-4})$alkyl, $(C_{1-4})$alkoxy, hydroxy, halogen or trifluoromethyl,

in free base or acid addition salt form.

3. A compound of formula I according to claim 1, wherein

$R_1$ is hydrogen or $(C_{1-4})$alkyl,

$R_2$ is hydrogen, chlorine, bromine or methyl,

$R_3$ is hydrogen, $(C_{1-4})$alkyl, benzyl optionally monosubstituted by $(C_{1-4})$alkoxy, or 2-furylmethyl,

$X_1$ is in position 10, 11 or 12 and is hydrogen, $(C_{1-4})$alkyl, $(C_{1-4})$alkoxy, hydroxy, halogen or trifluoromethyl and $X_2$ is hydrogen,

in free base or acid addition salt form.

4. A compound of formula I according to claim 1 from the group of the compounds of formula I in racemic form or with the indicated configuration, wherein $R_1$, $R_2$, $X_1$, $X_2$ and $R_3$ respectively are as follows:

H, H, H, H, ethyl,
H, H, H, H, n-propyl,
methyl, H, H, H, methyl,
H, H, H, H, allyl,
H, H, H, H, 2-furylmethyl,
H, H, H, H, p-methoxybenzyl,
H, H, 11-methoxy, H, methyl,
H, H, 11-hydroxy, H, methyl,
H, S, 10-methoxy, H, methyl,
H, H, 10-hydroxy, H, methyl,
H, H, 10-methyl, H, methyl,
H, H, 11-methyl, H, methyl,
H, H, 10-trifluoromethyl, H, methyl,
H, H, 10-methyl, 12-methyl, methyl,
H, H, 11-chloro, H, methyl,
H, H, 12-hydroxy, H, methyl,
H, H, H, H, methyl (+),
H, H, H, H, H,
H, H, 11-methoxy, H, H,
H, H, H, H, H (-),
methyl, H, H, H, methyl,
H, bromo, H, H, methyl,
H, chloro, H, H, methyl,
H, methyl, H, H, methyl,
H, bromo, H, H, methyl (-),
H, methyl, H, H, methyl (-),

in free base or acid addition salt form.

5. The (-)-trans-4,6,6a,7,8,12b-hexahydro-7-methylindolo[4,3-ab]phenanthridine in free base or acid addition salt form.

6. A process for the production of compounds of formula I according to claim 1 and their acid addition salts, characterized by oxidizing in 5,5a position of the indolophenanthridine ring compounds of formula II

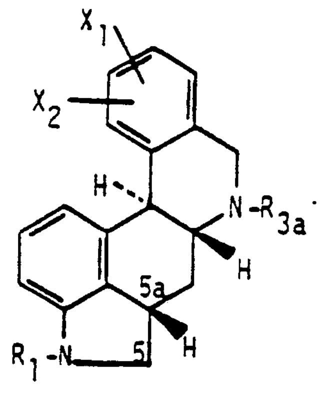

(II)

wherein

$R_1$, $X_1$ and $X_2$ are as defined in claim 1 and $R_3$, is as defined for $R_3$ in claim 1 but not hydrogen, if desired converting the resulting compounds of formula Ia,

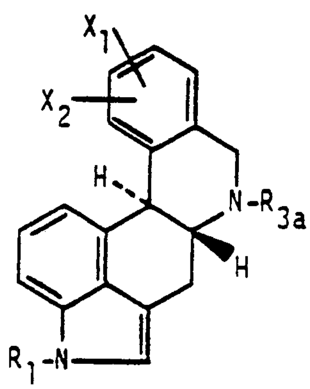

(Ia)

wherein

$R_1$, $R_{3a}$, $X_1$ and $X_2$ are as defined above, into further compounds of formula I and recovering the resulting compounds of formula I in free base or acid addition salt form.

7. A process for the production of compounds of formula Ib,

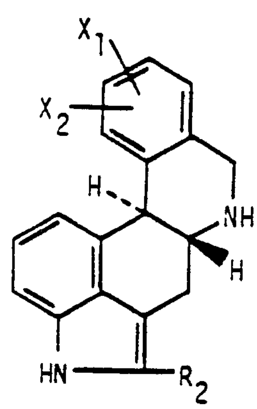

(Ib)

wherein

$R_2$, $X_1$ and $X_2$ are as defined in claim 1, and their acid addition salts, characterized by dealkylating in 7 position compounds of formula I',

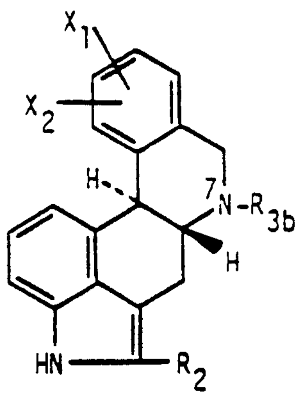

(I')

wherein

$R_2$, $X_1$ and $X_2$ are as defined in claim 1 and $R_{3b}$ is methyl or benzyl, and recovering the resultant compounds of formula Ib in free base or acid addition salt form.

8. A process for the production of compounds of formula Ic,

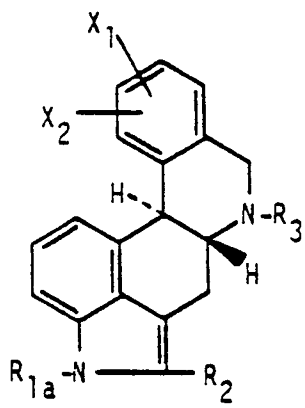

(Ic)

wherein

$R_2$, $R_3$, $X_1$ and $X_2$ are as defined in claim 1 and $R_{1a}$ is as defined for $R_1$ in claim 1 but not hydrogen, and their acid addition salts, characterized by alkylating in 4 position compounds of formula I'',

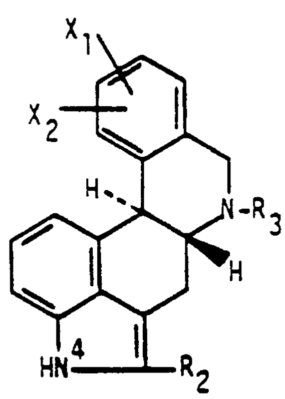

(I'')

wherein

$R_2$, $R_3$, $X_1$ and $X_2$ are as defined in claim 1, and recovering the resultant compounds of formula Ic in free base or acid addition salt form.

9. A process for the production of compounds of formula Id,

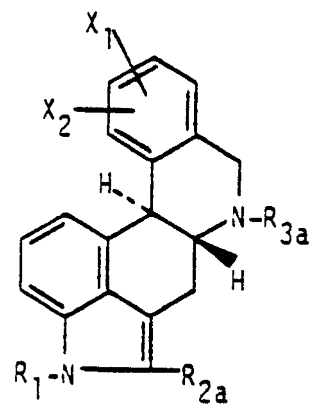

(Id)

wherein

$R_1$, $R_{3,}$, $X_1$ and $X_2$ are as defined in claims 1 and 6 and $R_{2,}$ is chlorine, bromine or methyl, and their acid addition salts, characterized by methylating or halogenating in 5 position compounds of formula Ia according to claim 6, and recovering the resultant compounds of formula Id in free base or acid addition salt form.

10. A compound according to any one of claims 1 to 5, in free base or acid addition salt form, for use as pharmaceutical.

11. A compound according to any one of claims 1 to 5, in free base or acid addition salt form, for the treatment of Parkinson's disease, cardiovascular diseases or impairing of the central nervous system, or for use as analgesic.

12. A pharmaceutical composition, including a compound according to any one of claims 1 to 5, in free base or pharmaceutically acceptable acid addition salt form.

13. A compound of formula II according to claim 6.

**Claims** for the Contracting State: AT

1. A process for the production of compounds of formula I,

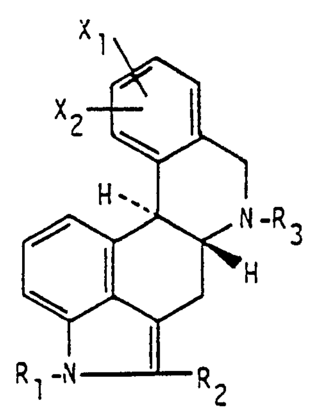

(I)

wherein

$R_1$ and $R_3$ independently are hydrogen, $(C_{1\text{-}4})$alkyl, $(C_{3\text{-}5})$alkenyl wherein the double bond is not adjacent to the nitrogen atom, $(C_{3\text{-}6})$cycloalkyl$(C_{1\text{-}3})$alkyl, phenyl$(C_{1\text{-}3})$alkyl optionally substituted in the phenyl ring by $(C_{1\text{-}4})$alkyl, $(C_{1\text{-}4})$alkoxy, hydroxy or halogen, or 2- or 3-furyl$(C_{1\text{-}3})$alkyl,

$R_2$ is hydrogen, chlorine, bromine or methyl,

$X_1$ and $X_2$ independently are hydrogen, $(C_{1\text{-}4})$alkyl, $(C_{1\text{-}4})$alkoxy, hydroxy, halogen or trifluoromethyl,

in free base or acid addition salt form, characterized by oxidizing in 5,5a position of the indolophenanthridine ring compounds of formula II,

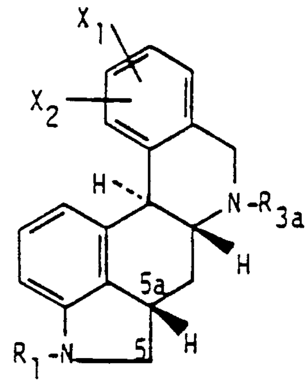

(II)

wherein

$R_1$, $X_1$ and $X_2$ are as defined above and $R_{3}$, is as defined for $R_3$ but not hydrogen, if desired converting the resulting compounds of formula Ia,

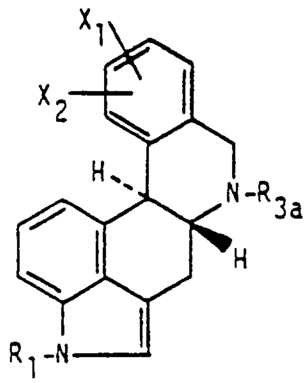

(Ia)

wherein

$R_1$, $R_{3a}$, $X_1$ and $X_2$ are as defined above, into further compounds of formula I and recovering the resulting compounds of formula I in free base or acid addition salt form.

2. A process for the production of compounds of formula Ib,

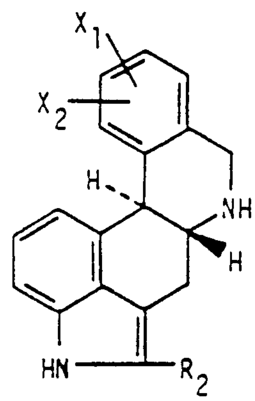

(Ib)

wherein

$R_2$, $X_1$ and $X_2$ are as defined in claim 1, and their acid addition salts, characterized by dealkylating in 7 position compounds of formula I',

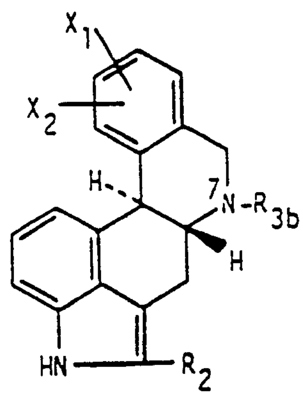

(I′)

wherein

$R_2$, $X_1$ and $X_2$ are as defined in claim 1 and $R_{3b}$ is methyl or benzyl, and recovering the resultant compounds of formula Ib in free base or acid addition salt form.

3. A process for the production of compounds of formula Ic,

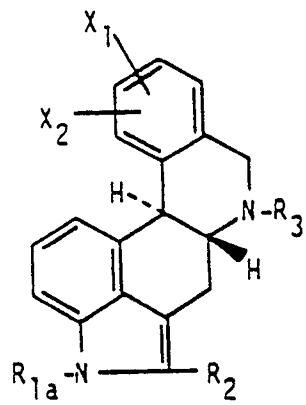

(Ic)

wherein

$R_2$, $R_3$, $X_1$ and $X_2$ are as defined in claim 1 and $R_{1a}$ is as defined for $R_1$ in claim 1 but not hydrogen, and their acid addition salts, characterized by alkylating in 4 position compounds of formula I″,

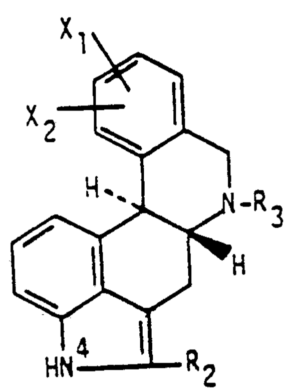

(I″)

wherein

$R_2$, $R_3$, $X_1$ and $X_2$ are as defined in claim 1, and recovering the resultant compounds of formula Ic in free base or acid addition salt form.

4. A process for the production of compounds of formula Id,

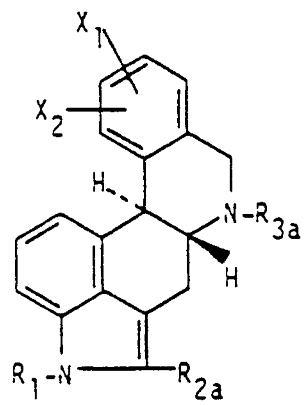

(Id)

wherein

$R_1$, $R_{3a}$, $X_1$ and $X_2$ are as defined in claim 1 and $R_{2a}$ is chlorine, bromine or methyl, and their acid addition salts, characterized by methylating or halogenating in 5 position compounds of formula Ia according to claim 1, and recovering the resultant compounds of formula Id in free base or acid addition salt form.

5. A process according to claim 1, for the production of compounds of formula I,
wherein
$R_1$ and $R_3$ independently are hydrogen, $(C_{1-4})$alkyl, $(C_{3-5})$alkenyl wherein the double bond is not adjacent to the nitrogen atom, $(C_{3-6})$cycloalkyl$(C_{1-3})$alkyl, phenyl$(C_{1-3})$alkyl optionally monosubstituted in the phenyl ring by methyl, methoxy, hydroxy or halogen, or 2- or 3-furyl$(C_1$-3)alkyl,
$R_2$ is hydrogen, chlorine, bromine or methyl,
$X_1$ and $X_2$ independently are hydrogen, $(C_{1-4})$alkyl, $(C_{1-4})$alkoxy, hydroxy, halogen or trifluoromethyl,
in free base or acid addition salt form.

6. A process according to claim 1, for the production of compounds of formula I, wherein
$R_1$ is hydrogen or $(C_1$-4)alkyl,
$R_2$ is hydrogen, chlorine, bromine or methyl,
$R_3$ is hydrogen, $(C_{1-4})$alkyl, benzyl optionally monosubstituted by $(C_{1-4})$alkoxy, or 2-furylmethyl,
$X_1$ is in position 10, 11 or 12 and is hydrogen, $(C_{1-4})$alkyl, $(C_{1-4})$alkoxy, hydroxy, halogen or trifluoromethyl and
$X_2$ is hydrogen,
in free base or acid addition salt form.

7. A process according to claim 1, for the production of compounds of formula I in racemic form or with the indicated configuration, wherein $R_1$, $R_2$, $X_1$, $X_2$ and $R_3$ respectively are as follows:
H, H, H, H, methyl,
H, H, H, H, ethyl,
H, H, H, H, n-propyl,
methyl, H, H, H, methyl,
H, H, H, H, allyl,
H, H, H, H, 2-furylmethyl,
H, H, H, H, p-methoxybenzyl,
H, H, 11-methoxy, H, methyl,
H, H, 11-hydroxy, H, methyl,
H, H, 10-methoxy, H, methyl,
H, H, 10-hydroxy, H, methyl,
H, H, 10-methyl, H, methyl,
H, H, 11-methyl, H, methyl,
H, H, 10-trifluoromethyl, H, methyl,
H, H, 10-methyl, 12-methyl, methyl,
H, H, 11-chloro, H, methyl,
H, H, 12-hydroxy, H, methyl,
H, H, H, H, methyl (+),
H, H, H, H, H,
H, H, 11-methoxy, H, H,
H, H, H, H, H (-),
methyl, H, H, H, methyl,
H, bromo, H, H, methyl,
H, chloro, H, H, methyl,
H, methyl, H, H, methyl,
H, bromo, H, H, methyl (-),
H, methyl, H, H, methyl (-),
in free base or acid addition salt form.

8. A process according to claim 1, for the production of the (-)-trans-4,6,6a,7,8,12b-hexahydro-7-methyl-indolo[4,3-ab]phenanthridine in free base or acid addition salt form.

9. A 4,6,6a,7,8,12b-hexahydro-indolo[4,3-ab]phenanthridine in free base or acid addition salt form, whenever produced according to any one of claims 1 to 8.

10. A pharmaceutical composition including a compound according to claim 9, in free base or pharmaceutically acceptable acid addition salt form.

11. A process for the production of a pharmaceutical composition, characterized by mixing a compound of formula I,

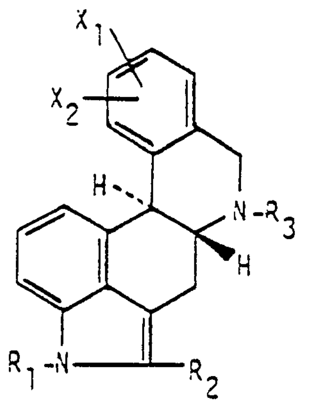

(I)

wherein

$R_1$ and $R_3$ independently are hydrogen, $(C_{1-4})$alkyl, $(C_{3}$-5)alkenyl wherein the double bond is not adjacent to the nitrogen atom, $(C_{3-6})$cycloalkyl$(C_{1-3})$alkyl, phenyl$(C_{1-3})$alkyl optionally substituted in the phenyl ring by $(C_{1-4})$alkyl, $(C_{1-4})$alkoxy, hydroxy or halogen, or 2- or 3-furyl$(C_{1-3})$alkyl,

$R_2$ is hydrogen, chlorine, bromine or methyl,

$X_1$ and $X_2$ independently are hydrogen, $(C_{1-4})$alkyl, $(C_{1-4})$alkoxy, hydroxy, halogen or trifluoromethyl,

in free base or pharmaceutically acceptable acid addition salt form, with pharmaceutically acceptable diluents or carriers.

12. A process for the production of compounds of formula II according to claim 1, characterized by reducing and optionally alkylating in position 4 compounds of formula III,

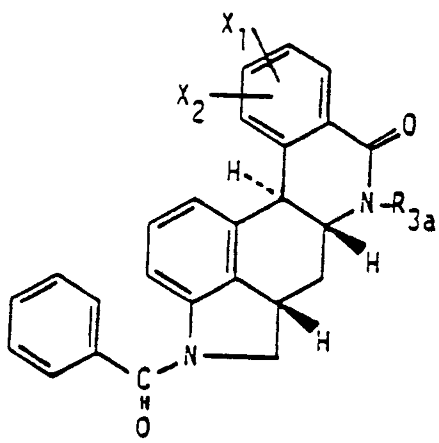

(III)

wherein

$R_{3a}$, $X_1$ and $X_2$ are as defined in claim 1.

**Revendications** pour les Etats Contractants: BE, CH, DE, FR, GB, IT, LI, LU, NL, SE

1. Un composé de formule I

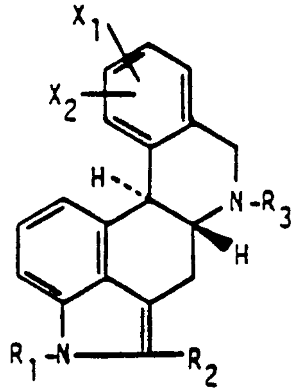

I

dans laquelle

$R_1$ et $R_3$ signifient chacun, indépendamment l'un de l'autre, l'hydrogène, un groupe alkyle en $C_1$-$C_4$, alcényle en $C_3$-$C_5$, la double liaison n'étant pas adjacente à l'atome d'azote, (cycloalkyl en $C_3$-$C_6$)-alkyle en $C_1$-$C_3$, phényl-alkyle en $C_1$-$C_3$ éventuellement substitué dans le cycle phényle par de l'halogène ou par des groupes alkyle en $C_1$-$C_4$, alcoxy en $C_1$-$C_4$ ou hydroxy, ou un groupe 2- ou 3-furyl-alkyle en $C_1$-$C_3$,

$R_2$ signifie l'hydrogène, le chlore, le brome ou un groupe méthyle, et

$X_1$ et $X_2$ signifient chacun, indépendamment l'un de l'autre, l'hydrogène, un halogène ou un groupe alkyle en $C_1$-$C_4$, alcoxy en $C_1$-$C_4$, hydroxy ou trifluorométhyle,

sous forme de base libre ou d'un sel d'addition d'acide.

2. Un composé de formule I selon la revendication 1,

dans lequel

$R_1$ et $R_3$ signifient chacun, indépendamment l'un de l'autre, l'hydrogène, un groupe alkyle en $C_1$-$C_4$, alcényle en $C_3$-$C_5$, la double liaison n'étant pas adjacente à l'atome d'azote, (cycloalkyl en $C_3$-$C_6$)-alkyle en $C_1$-$C_3$, phényl-alkyle en $C_1$-$C_3$ éventuellement monosubstitué dans le cycle phényle par un halogène ou par un groupe méthyle, méthoxy ou hydroxy, ou un groupe 2- ou 3-furyl-alkyle en $C_1$-$C_3$,

$R_2$ signifie l'hydrogène, le chlore, le brome ou un groupe méthyle, et

$X_1$ et $X_2$ signifient chacun, indépendamment l'un de l'autre, l'hydrogène, un halogène ou un groupe alkyle en $C_1$-$C_4$, alcoxy en $C_1$-$C_4$, hydroxy ou trifluorométhyle,

sous forme de base libre ou d'un sel d'addition d'acide.

3. Un composé de formule I selon la revendication 1,

dans lequel

$R_1$ signifie l'hydrogène ou un groupe alkyle en $C_1$-$C_4$,

$R_2$ signifie l'hydrogène, le chlore, le brome ou un groupe méthyle,

$R_3$ signifie l'hydrogène, un groupe alkyle en $C_1$-$C_4$, un groupe benzyle éventuellement monosubstitué par un groupe alcoxy en $C_1$-$C_4$ ou un groupe 2-furyl-méthyle,

$X_1$ situé en position 10, 11 ou 12, signifie l'hydrogène, un halogène ou un groupe alkyle en $C_1$-$C_4$, alcoxy en $C_1$-$C_4$, hydroxy ou trifluorométhyle, et

$X_2$ signifie l'hydrogène,

sous forme de base libre ou d'un sel d'addition d'acide.

4. Un composé de formule I selon la revendication 1 choisi dans le groupe descomposés de formule I sous forme racémique ou avec la configuration indiquée, dans lesquels $R_1$, $R_2$, $X_1$, $X_2$ et $R_3$ ont respectivement les significations suivantes:

H H H, H, éthyle,
H H H H, n-propyle,
méthyle, H, H, H, méthyle,
H, H, H, H, allyle,
H, H, H, H, 2-furylméthyle,
H, H, H, H, p-méthoxybenzyle,
H, H, 11-méthoxy, H, méthyle,
H, H, 11-hydroxy, H, méthyle,
H, H, 10-méthoxy, H, méthyle,
H, H, 10-hydroxy, H, méthyle,
H, H, 10-méthyle, H, méthyle,
H, H, 11-méthyle, H, méthyle,
H, H, 10-trifluorométhyle, H, méthyle,
H, H, 10-méthyle, 12-méthyle, méthyle,
H, H, 11-chloro, H, méthyle,
H, H, 12-hydroxy, H, méthyle,
H, H, H, H, méthyle (+),

H, H, H, H, H,
H, H, 11-méthoxy, H, H,
H, H, H, H, H (-),
méthyle, H, H, H, méthyle,
H, bromo, H, H, méthyle,
H, chloro, H, H, méthyle,
H, méthyle, H, H, méthyle,
H, bromo, H, H, méthyle (-),
H, méthyle, H, H, méthyle (-),
sous forme de base libre ou d'un sel d'addition d'acide.

5. La (-)-trans-4,6,6a,7,8,12b-hexahydro-7-méthyl-indolo[4,3-ab]phénanthridine sous forme de base libre ou d'un sel d'addition d'acide.

6. Procédé de préparation des composés de formule I selon la revendication 1 et de leurs sels d'addition d'acides, caractérisé en ce qu'on oxyde en position 5,5a du squelette de l'indolophénanthridine des composés de formule II

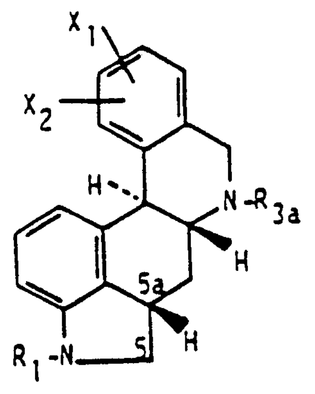

II

dans laquelle
$R_1$, $X_1$ et $X_2$ sont tels que spécifiés à la revendication 1 et $R_{3a}$ la signification donnée pour $R_3$ à la revendication 1 sauf l'hydrogène, on transforme si on le désire les composés obtenus de formule Ia

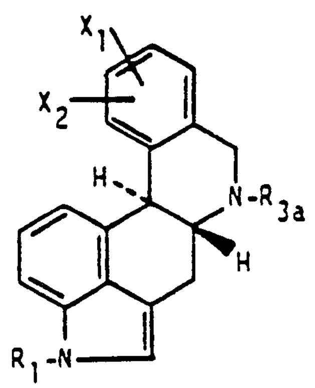

Ia

dans laquelle
$R_1$, $R_{3a}$, $X_1$ et $X_2$ ont les significations indiquées plus haut, en d'autres composés de formule I, et on récupère les composés obtenus de formule I sous forme de bases libres ou de sels d'addition d'acides.

7. Procédé de préparation des composés de formule Ib

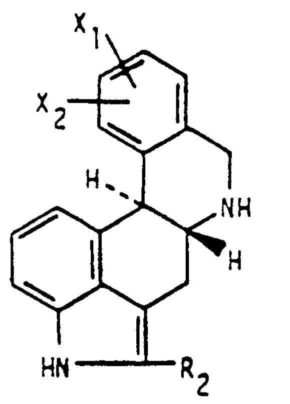

Ib

dans laquelle

$R_2$, $X_1$ et $X_2$ sont tels que spécifiés à la revendication 1, et de leurs sels d'addition d'acides, caractérisé en ce qu'on désalkyle en position 7 des composés de formule I'

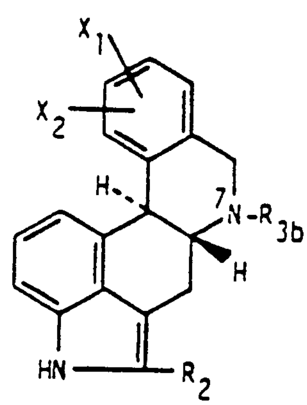

I'

dans laquelle

$R_2$, $X_1$ et $X_2$ sont tels que spécifiés à la revendication 1 et $R_{3b}$ signifie un groupe méthyle ou benzyle, et on récupère les composés obtenus de formule Ib sous forme de bases libres ou de sels d'addition d'acides.

8. Procédé de préparation des composés de formule Ic

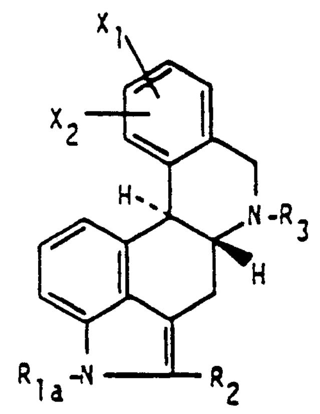

Ic

dans laquelle

$R_2$, $R_3$, $X_1$ et $X_2$ sont tels que spécifiés à la revendication 1 et $R_{1a}$, a la signification donnée pour $R_1$ à la revendication 1 à l'exception de l'hydrogène, et de leurs sels d'addition d'acides, caractérisé en ce qu'on alkyle en position 4 des composés de formule I''

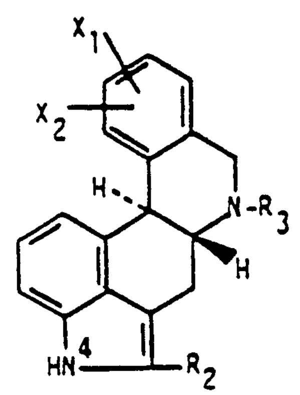

I''

dans laquelle

$R_2$, $R_3$, $X_1$ et $X_2$ sont tels que spécifiés à la revendication 1, et on récupère les composés obtenus de formule Ic sous forme de bases libres ou de sels d'addition d'acides.

9. Procédé de préparation des composés de formule Id

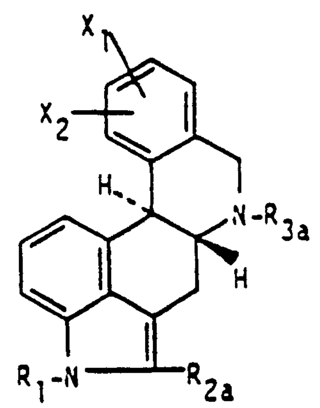

Id

dans laquelle

$R_1$, $R_{3a}$, $X_1$ et $X_2$ sont tels que spécifiés aux revendications 1 et 6 et $R_{2a}$ signifie le chlore, le brome ou un groupe méthyle, et de leurs sels d'addition d'acides, caractérisé en ce qu'on méthyle ou qu'on halogène en position 5 les composés de formule Ia selon la revendication 6, et on récupère les composés obtenus de formule Id sous forme de bases libres ou de sels d'addition d'acides.

10. Un composé selon l'une quelconque des revendications 1 à 5, sous forme libre ou sous forme d'un sel d'addition d'acide pharmaceutiquement acceptable, pour l'utilisation comme médicament.

11. Un composé selon l'une quelconque des revendications 1 à 5, sous forme libre ou sous forme d'un sel d'addition d'acide pharmaceutiquement acceptable, pour le traitement de la maladie de Parkinson, des

maladies du système cardiovasculaire ou des altérations du système nerveux central, ou pour l'utilisation comme analgésique.

12. Une composition pharmaceutique, comprenant un composé selon l'une quelconque des revendications 1 à 5, sous forme libre ou sous forme d'un sel d'addition d'acide pharmaceutiquement acceptable.

13. Un composé de formule II selon la revendication 6.

**Revendications** pour l'Etat Contractant: AT

1. Procédé de préparation des composés de formule I

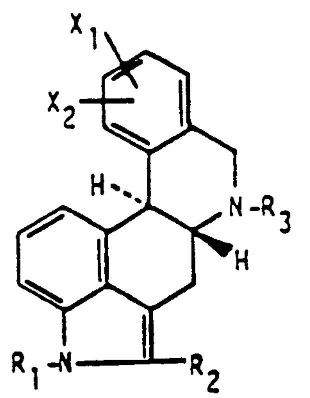

I

dans laquelle

$R_1$ et $R_3$ signifient chacun, indépendamment l'un de l'autre, l'hydrogène, un groupe alkyle en $C_1$-$C_4$, alcényle en $C_3$-$C_5$, la double liaison n'étant pas adjacente à l'atome d'azote, (cycloalkyl en $C_3$-$C_6$)-alkyle en $C_1$-$C_3$, phényl-alkyle en $C_1$-$C_3$ éventuellement substitué dans le cycle phényle par de l'halogène ou par des groupes alkyle en $C_1$-$C_4$, alcoxy en $C_1$-$C_4$ ou hydroxy, ou un groupe 2- ou 3-furyl-alkyle en $C_1$-$C_3$,

$R_2$ signifie l'hydrogène, le chlore, le brome ou un groupe méthyle, et

$X_1$ et $X_2$ signifient chacun, indépendamment l'un de l'autre, l'hydrogène, un halogène ou un groupe alkyle en $C_1$-$C_4$, alcoxy en $C_1$-$C_4$, hydroxy ou trifluorométhyle,

sous forme de bases libres ou de sels d'addition d'acides, caractérisé en ce qu'on oxyde en position 5,5a du squelette de l'indolophénanthridine des composés de formule II

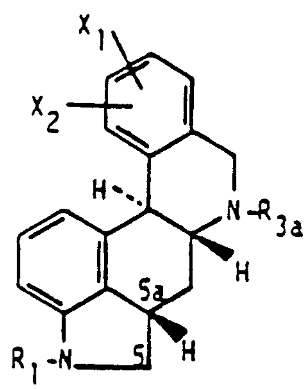

II

dans laquelle

$R_1$, $X_1$ et $X_2$ sont tels que spécifiés plus haut et $R_{3a}$ a la signification donnée pour $R_3$ sauf l'hydrogène, on transforme si on le désire les composés obtenus de formule Ia

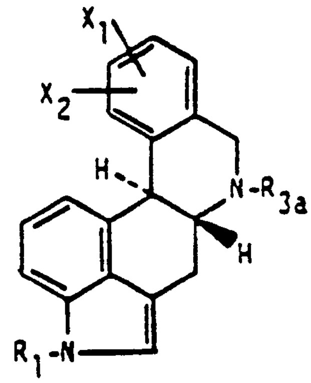

Ia

dans laquelle

$R_1$, $R_{3a}$, $X_1$ et $X_2$ ont les significations indiquées plus haut, en d'autres composés de formule I, et on récupère les composés obtenus de formule I sous forme de bases libres ou de sels d'addition d'acides.

2. Procédé de préparation des composés de formule Ib

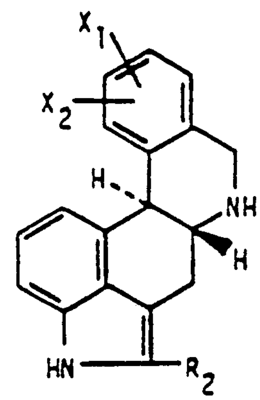

Ib

dans laquelle

$R_2$, $X_1$ et $X_2$ sont tels que spécifiés à la revendication 1, et de leurs sels d'addition d'acides, caractérisé en ce qu'on désalkyle en position 7 des composés de formule I'

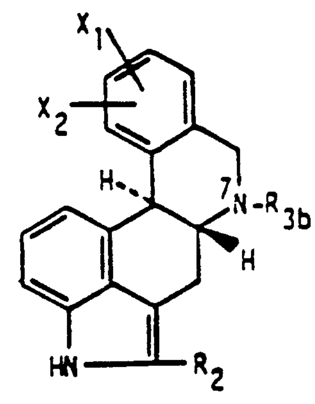

I'

dans laquelle

$R_2$, $X_1$ et $X_2$ sont tels que spécifiés à la revendication 1 et $R_{3b}$ signifie un groupe méthyle ou benzyle, et on récupère les composés obtenus de formule Ib sous forme de bases libres ou de sels d'addition d'acides.

3. Procédé de préparation des composés de formule Ic

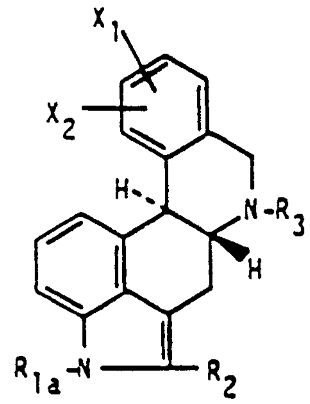

Ic

dans laquelle

$R_2$, $R_3$, $X_1$ et $X_2$ sont tels que spécifiés à la revendication 1 et $R_{1a}$ a la signification donnée pour $R_1$ à la revendication 1 à l'exception de l'hydrogène, et de leurs sels d'addition d'acides, caractérisé en ce qu'on alkyle en position 4 des composés de formule I''

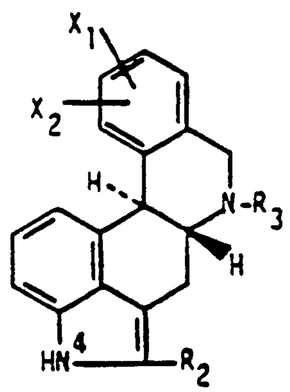

I''

dans laquelle

$R_2$, $R_3$, $X_1$ et $X_2$ sont tels que spécifiés à la revendication 1, et on récupère les composés obtenus de formule Ic sous forme de bases libres ou de sels d'addition d'acides.

4. Procédé de préparation des composés de formule Id

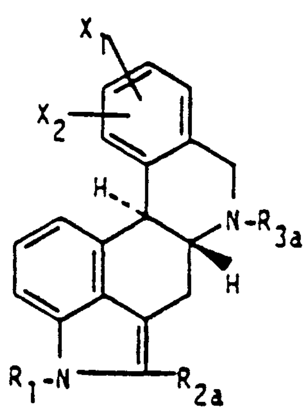

Id

dans laquelle

$R_1$, $R_{3a}$, $X_1$ et $X_2$ sont tels que spécifiés à la revendication 1 et $R_{2a}$ signifie le chlore, le brome ou un groupe méthyle, et de leurs sels d'addition d'acides, caractérisé en ce qu'on méthyle ou qu'on halogène en position 5 les composés de formule Ia selon la revendication 1, et on récupère les composés obtenus de formule Id sous forme de bases libres ou de sels d'addition d'acides.

5. Procédé selon la revendication 1, pour la préparation des composés de formule I, dans lesquels

$R_1$ et $R_3$ signifient chacun, indépendamment l'un de l'autre, l'hydrogène, un groupe alkyle en $C_1$-$C_4$, alcényle en $C_3$-$C_5$, la double liaison n'étant pas adjacente à l'atome d'azote, (cycloalkyl en $C_3$-$C_6$)-alkyle en $C_1$-$C_3$, phénylalkyle en $C_1$-$C_3$ éventuellement monosubstitué dans le cycle phényle par un halogène ou par un groupe

méthyle, méthoxy ou hydroxy, ou un groupe 2- ou 3-furyl-alkyle en $C_1$-$C_3$,

$R_2$ signifie l'hydrogène, le chlore, le brome ou un groupe méthyle, et

$X_1$ et $X_2$ signifient chacun, indépendamment l'un de l'autre, l'hydrogène, un halogène ou un groupe alkyle en $C_1$-$C_4$, alcoxy en $C_1$-$C_4$, hydroxy ou trifluorométhyle,

sous forme de bases libres ou de sels d'addition d'acides.

6. Procédé selon la revendication 1, pour la préparation des composés de formule I, dans lesquels

$R_1$ signifie l'hydrogène ou un groupe alkyle en $C_1$-$C_4$,

$R_2$ signifie l'hydrogène, le chlore, le brome ou un groupe méthyle,

$R_3$ signifie l'hydrogène, un groupe alkyle en $C_1$-$C_4$, un groupe benzyle éventuellement monosubstitué par un groupe alcoxy en $C_1$-$C_4$ ou un groupe 2-furylméthyle,

$X_1$ est situé en position 10, 11 ou 12 et signifie l'hydrogène, un halogène ou un groupe alkyle en $C_1$-$C_4$, alcoxy en $C_1$-$C_4$, hydroxy ou trifluorométhyle, et

$X_2$ signifie l'hydrogène,

sous forme de bases libres ou de sels d'addition d'acides.

7. Procédé selon la revendication 1, pour la préparation des composés de formule I sous forme racémique ou avec la configuration indiquée, dans lesquels $R_1$, $R_2$, $X_1$, $X_2$ et $R_3$ ont respectivement les significations suivantes:

H, H, H, H, méthyle,
H, H, H, H, éthyle,
H, H, H, H, n-propyle,
méthyle, H, H, H, méthyle,
H, H, H, H, allyle,
H, H, H, H, 2-furylméthyle,
H, H, H, H, p-méthoxybenzyle,
H, H, 11-méthoxy, H, méthyle,
H, H, 11-hydroxy, H, méthyle,
H, H, 10-méthoxy, H, méthyle,
H, H, 10-hydroxy, H, méthyle,
H, H, 10-méthyle, H, méthyle,
H, H, 11-méthyle, H, méthyle,
H, H, 10-trifluorométhyle, H, méthyle,
H, H, 10-méthyle, 12-méthyle, méthyle,
H, H, 11-chloro, H, méthyle,
H, H, 12-hydroxy, H, méthyle,
H, H, H, H, méthyle (+),
H, H, H, H, H,
H, H, 11-méthoxy, H, H,
H, H, H, H, H (-),
méthyle, H, H, H, méthyle,
H, bromo, H, H, méthyle,
H, chloro, H, H, méthyle,
H, méthyle, H, H, méthyle,
H, bromo, H, H, méthyle (-),
H, méthyle, H, H, méthyle (-),

sous forme de bases libres ou de sels d'addition d'acides.

8. Procédé selon la revendication 1 pour la préparation de la (-)-trans-4,6,6a,7,8,12b-hexahydro-7-méthyl-indolo[4,3-ab]phénanthridine sous forme de base libre ou d'un sel d'addition d'acide.

9. Une 4,6,6a,7,8,12b-hexahydro-indolo[4,3-ab]phénanthridine sous forme de base libre ou d'un sel d'addition d'acide, préparée selon l'une quelconque des revendications 1 à 8.

10. Une composition pharmaceutique, comprenant un composé selon la revendication 9, sous forme libre ou sous forme d'un sel d'addition d'acide pharmaceutiquement acceptable.

11. Un procédé de préparation d'une composition pharmaceutique, caractérisé en ce qu'on mélange un composé de formule I

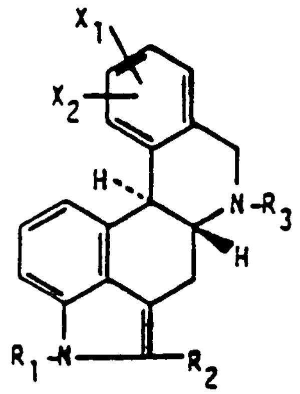

I

dans laquelle

$R_1$ et $R_3$ signifient chacun, indépendamment l'un de l'autre, l'hydrogène, un groupe alkyle en $C_1$-$C_4$, alcényle en $C_3$-$C_5$, la double liaison n'étant pas adjacente à l'atome d'azote, (cycloalkyl en $C_3$-$C_6$)-alkyle en $C_1$-$C_3$, phényl-alkyle en $C_1$-$C_3$ éventuellement substitué dans le cycle phényle par de l'halogène ou par des groupes alkyle en $C_1$-$C_4$, alcoxy en $C_1$-$C_4$ ou hydroxy, ou un groupe 2- ou 3-furyl-alkyle en $C_1$-$C_3$,

$R_2$ signifie l'hydrogène, le chlore, le brome ou un groupe méthyle, et

$X_1$ et $X_2$ signifient chacun, indépendamment l'un de l'autre, l'hydrogène, un halogène ou un groupe alkyle en $C_1$-$C_4$, alcoxy en $C_1$-$C_4$, hydroxy ou trifluorométhyle,

sous forme libre ou sous forme d'un sel d'addition d'acide pharmaceutiquement acceptable, avec des diluants ou véhicules pharmaceutiquement acceptables.

12. Procédé de préparation des composés de formule II selon la revendication 1, caractérisé en ce qu'on réduit des composés de formule III

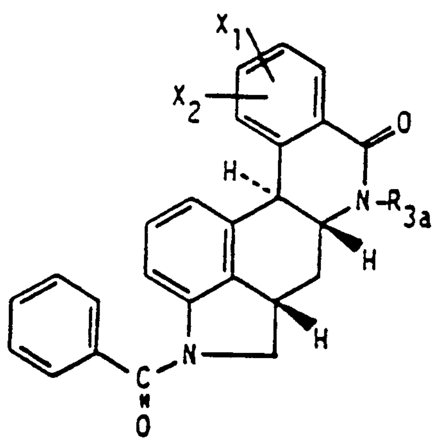

III

dans laquelle

$R_{3a}$, $X_1$ et $X_2$ sont tels que spécifiés à la revendication 1, et éventuellement on alkyle en position 4.